(19) Europäisches Patentamt
European Patent Office
Office européen des brevets

(11) Veröffentlichungsnummer : **0 189 045**
**B1**

# (12) EUROPÄISCHE PATENTSCHRIFT

(45) Veröffentlichungstag der Patentschrift : **14.02.90**

(51) Int. Cl.[5] : **C 07 D471/04**, A 61 K 31/505 // (C07D471/04, 239:00, 221:00)

(21) Anmeldenummer : **86100096.6**

(22) Anmeldetag : **07.01.86**

(54) **Pyridopyrimidine, mehrere Verfahren zu ihrer Herstellung sowie ihre Verwendung als Arzneimittel.**

(30) Priorität : **19.01.85 DE 3501696**

(43) Veröffentlichungstag der Anmeldung : **30.07.86 Patentblatt 86/31**

(45) Bekanntmachung des Hinweises auf die Patenterteilung : **14.02.90 Patentblatt 90/07**

(84) Benannte Vertragsstaaten : **AT BE CH DE FR GB IT LI NL SE**

(56) Entgegenhaltungen :
US--A-- 3 021 332
US--A-- 3 505 332

(73) Patentinhaber : **BAYER AG**
**D-5090 Leverkusen 1 Bayerwerk (DE)**

(72) Erfinder : **Meyer, Horst, Dr.**
**Henselweg 11**
**D-5600 Wuppertal 1 (DE)**
Erfinder : **Wehinger, Egbert**
**Gellertweg 33**
**D-5600 Wuppertal 1 (DE)**
Erfinder : **Garthoff, Bernward, Dr.**
**Händelstrasse 22**
**D-4010 Hilden (DE)**
Erfinder : **Kazda, Stanislav, Dr.**
**Gellertweg 18**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft neue Pyridopyrimidine, mehrere Verfahren zu ihrer Herstellung sowie ihre Verwendung als Arzneimittel, insbesondere in kreislaufbeeinflussenden Mitteln.

Es ist bereits bekannt geworden, daß man Derivate des 1.2.3.7-Tetrahydro-8-(2-imidazolin-2-yl)-imidazo [1.2-a]-pyridins erhält, wenn man äquimolare Mengen eines Aldehyds und einer β-Dicarbonylverbindung mit dem Hydrochlorid des 2.2'-Methylendiimidazolins zur Reaktion bringt (H. Meyer, Liebigs Ann. Chem. 1981, 1523).

In dem US-Patent 3 505 332 werden einige 2,4,7-Triamino-pyridopyrimidine mit diuretischer Wirkung beschrieben. Diese unterscheiden sich strukturell eindeutig von den erfindungsgemäßen Verbindungen. Insbesondere durch die Substitution in 7-Stellung (Substituent $R_3$) und 6-Stellung (—$COR_2$-Gruppe).

Gegenstand der vorliegenden Erfindung sind neue Pyrdopyrimidine der allgemeinen Formel I

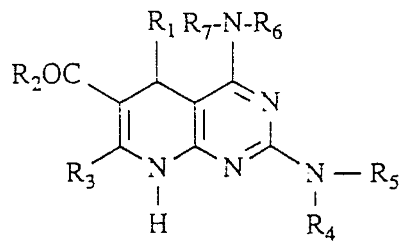

in welcher

$R_1$ für einen Phenyl- oder Naphthylrest oder für Thienyl Furyl Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisooxazolyl, Benzoxadiazolyl, Benzthiadiazolyl, Chinolyl, Isochinolyl, Chinazolyl oder Chinoxalyl steht, wobei die genannten Heterocyclen sowie der Phenylrest und der Naphthylrest jeweils 1 bis 5 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest, Carbamoyl, N.N-Dimethylcarbamoyl, Sulfamoyl, $SO_m$-Alkyl, worin m 0 bis 2 bedeutet und Alkyl 1 bis 4 Kohlenstoffatome enthält, oder $SO_m$-Benzyl mit m = 0 bis 2 aufgeführt seien,

$R_2$ für einen geradkettigen, verzweigten oder cyclischen Alkylrest mit bis zu 8 Kohlenstoffatomen oder für eine Phenyl- oder Benzylgruppe steht,

oder

für eine Amino-, Mono- oder Dialkylaminogruppe mit bis zu 4 Kohlenstoffatomen je Alkylgruppe steht, wobei eine oder beide Alkylgruppen durch einen Phenylrest substituiert sein können,

oder

für einen Anilinorest steht, der gegebenenfalls durch Fluor oder Chlor, oder durch Alkyl oder Alkoxy mit 1 bis 4 Kohlenstoffatomen substituiert ist,

oder

für den Rest $OR_8$ steht, wobei $R_8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Benzoyloxy, Nitro, Nitrooxy, Alkoxycarbonyl mit bis zu 4 Kohlenstoffatomen in der Alkoxygruppe, Phenyl, Phenoxy, Phenylthio, Phenylsulfonyl, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 Kohlenstoffatomen, Alkoxyalkyl mit bis zu 6 Kohlenstoffatomen, Phenyl, Benzyl oder Phenethyl trägt oder wobei der Stickstoff dieser Aminogruppe gegebenenfalls mit den Substituenten einen 5- bis 7-gliedrigen Ring bildet, die als weiteres Heteroatom ein Sauerstoff- oder Schwefelatom oder eine N-Phenylgruppe oder eine N-Alkylgruppe mit 1 bis 4 Kohlenstoffatomen im Alkylrest enthalten kann,

$R_3$ für Wasserstoff steht

oder

für einen geradkettigen, verzweigten oder cyclischen Kohlenwasserstoffrest mit bis zu 6 Kohlenstoffatomen steht, der gegebenenfalls durch 1 oder 2 Alkoxygruppen mit 1 bis 4 Kohlenstoffatomen oder eine Acetoxy- oder Benzoyloxygruppe substituiert ist,

oder

für einen Phenylrest oder einen Benzylrest steht,

und

$R_4$, $R_5$, $R_6$ und $R_7$ gleich oder verschieden sind und jeweils für Wasserstoff stehen
oder
für einen gegebenenfalls durch Hydroxy, Methoxy, Acetoxy oder Benzoyloxy substituierten geradkettigen oder verzweigten Alkylrest mit bis zu 8 Kohlenstoffatomen stehen, wobei die Reste $R_4$, $R_5$ sowie $R_6$ und $R_7$ mit dem jeweiligen Stickstoffatom einen 5 bis 7 gliedrigen Ring bilden können, der gegebenenfalls als weiteres Heteroatom Sauerstoff oder Schwefel oder eine N-Alkylgruppe mit bis zu 4 Kohlenstoffatomen oder eine N-Phenylgruppe enthält,
oder
für einen Phenyl- oder Benzylrest stehen.

Es wurde gefunden,. daß man die erfindungsgemäßen Pyridopyrimidine der allgemeinen Formel I erhält, wenn man

A) Yliden-β-dicarbonylverbindungen der allgemeinen Formel (II)

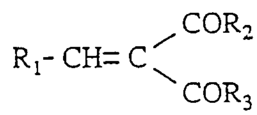

in welcher
$R_1$, $R_2$ und $R_3$ die oben angegebene Bedeutung haben mit 6-Aminopyrimidinen der allgemeinen Formel III,

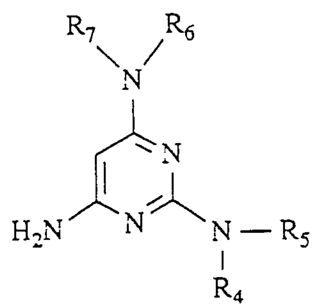

in welcher
$R_4$, $R_5$, $R_6$ und $R_7$ die oben angegebene Bedeutung haben, gegebenenfalls in Gegenwart organischer Lösungsmittel umsetzt, oder

B) Aldehyde der allgemeinen Formel IV

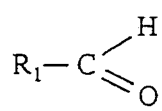

in welcher
$R_1$ die oben angegebene Bedeutung hat, mit β-Dicarbonylverbindungen der allgemeinen Formel V

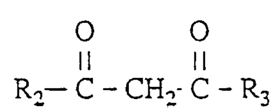

in welcher
$R_2$ und $R_3$ die oben angegebene Bedeutung haben, und 6-Aminopyrimidinen der allgemeinen Formel III

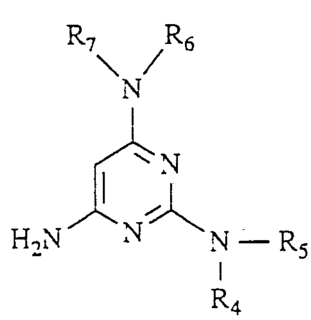

in welcher

$R_4$, $R_5$, $R_6$ und $R_7$ die oben angegebene Bedeutung haben, gegebenenfalls in Gegenwart organischer Lösungsmittel zur Reaktion bringt.

Die erfindungsgemäßen Pyrdopyrimidine der allgemeinen Formel I sind neu und besitzen wertvolle pharmakologische Eigenschaften. Aufgrund ihrer kreislaufbeeinflussenden, renal vasodilatierenden und diuretischer Wirkung können sie als antihypertensive Mittel, als Vasodilatatoren und als Diuretika bei verschiedenen Störungen des Kreislaufs und des Elektrolythaushalts Verwendung finden und sind somit als Bereicherung der Pharmazie anzusehen.

Durch die basischen Funktionen können die erfindungsgemäßen Verbindungen in Abhängigkeit des Herstellungsverfahrens als Salze anfallen. Sowohl die freien Basen als auch die Salze der Pyridopyrimidine der allgemeinen Formel I sind Gegenstand der vorliegenden Erfindung.

Je nach Art der verwendeten Ausgangsstoffe kann die Synthese der erfindungsgemäßen Verbindungen durch folgende Formelschemata wiedergegeben werden, wobei die Herstellung von 2.4-Diamino-7-methyl-5-(2-nitrophenyl-5.8-dihydro-pyrido [2.3-d] pyrimidin-6-carbonsäureisobutylester sowie von 2.4-Dimorpholino-7-methyl-5-(3-trifluormethylphenyl)-5.8-dihydro-pyrido [2.3-d]-pyrimidin-6-carbonsäureethylester als Beispiele gewählt seien

A)

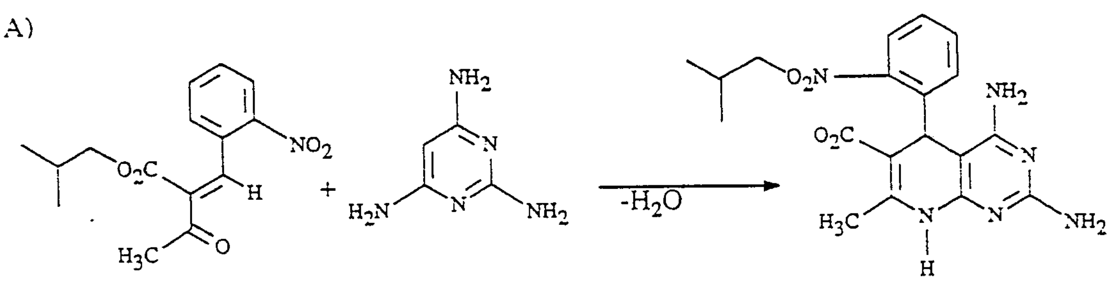


B)

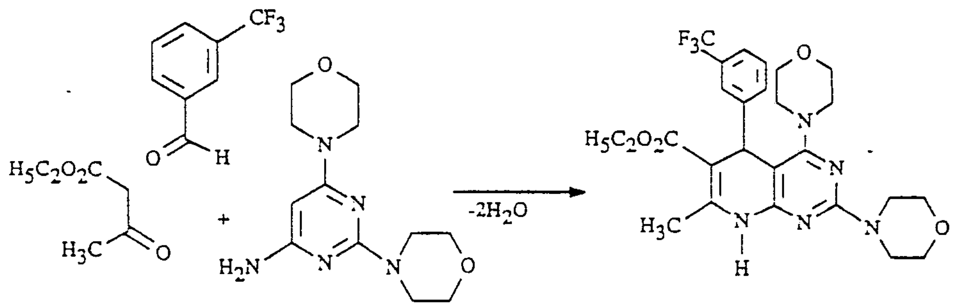


Verfahrensvariante A

Gemäß Verfahren A wird eine Yliden-β-dicarbonylverbindung der allgemeinen Formel II

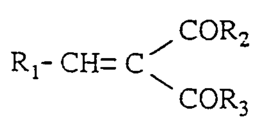

mit einem 6-Aminopyrimidin der allgemeinen Formel III

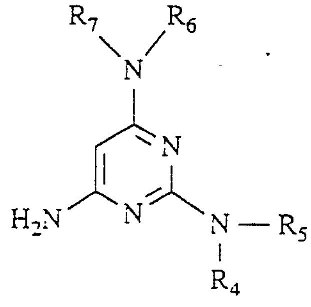

zur Reaktion gebracht.

In den Formeln I und II steht vorzugsweise

$R_1$ für einen Phenyl- oder Naphthylrest oder für Thienyl, Furyl, Pyridyl, Pyridazinyl, Pyrimidyl, Benzoxadiazolyl, Benzthiadiazolyl, Chinolyl oder Isochinolyl steht, wobei die genannten Heterocyclen sowie der Phenyl- und der Naphthylrest 1 bis 5 gleiche oder verschiedene Substituenten tragen können, aus der Gruppe geradkettiges oder verzweigtes Alkyl mit bis zu 8 Kohlenstoffatomen, Alkoxyalkyl mit 1 bis 4 Kohlenstoffatomen, Dioxymethylen, Fluor, Chlor, Brom, Trifluormethyl, Trifluormethoxy, Difluormethoxy, Nitro, Cyano, Azido, Thioalkyl, Sulfonylalkyl mit jeweils 1 bis 4 C-Atomen im Alkylrest, Thiobenzyl und Sulfonylbenzyl,

$R_2$ für Alkyl mit bis zu 8 C-Atomen, Phenyl oder Benzyl steht,

oder

für den Rest $OR_8$ steht, wobei $R_8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Phenyl, Phenoxy, Phenylthio, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 C-Atomen, Phenyl oder Benzyl trägt,

$R_3$ für einen geradkettigen oder verzweigten Kohlenwasserstoffrest mit bis zu 6 C-Atomen steht, der gegebenenfalls substituiert ist durch 1 oder 2 Alkoxygruppen mit 1 bis 4 C-Atomen oder eine Acetoxygruppe,

oder

für einen Phenylrest oder Benzylrest steht, und

$R_4$, $R_5$, $R_6$ und $R_7$ jeweils gleich oder verschieden sind und für Wasserstoff oder einen geradkettigen oder verzweigten Alkylrest mit bis zu 8 Kohlenstoffatomen stehen, der gegebenenfalls substituiert ist durch Hydroxy, Methoxy oder Acetoxy und wobei die Reste $R_4$ und $R_5$ sowie $R_6$ und $R_7$ mit dem jeweiligen Stickstoffatomen einen 5 bis 7 gliedrigen Ring bilden können, der gegebenenfalls als weiteres Heteroatom Sauerstoff, Schwefel oder eine N-Alkylgruppe mit bis zu 4 C-Atomen oder eine N-Phenylgruppe enthält.

Die als Ausgangsstoffe verwendeten Yliden-β-dicarbonylverbindungen der Formel II sind literaturbekannt oder können nach literaturbekannten Methoden hergestellt werden (vgl. z. B. G. Jones « The Knoevenagel Condensation » in Org. Reactions, Vol. XV, 204 f (1967)).

Als Beispiele seien genannt :

Benzylidenacetylaceton, β,β-Dibenzoylstyrol, 2'-Nitrobenzylidenacetylaceton, 3'-Nitrobenzylidenacetessigsäureanilid, 3'-Nitrobenzylidenacetessigsäureamid, 3'-Nitrobenzylidenacetessigsäuredimethylamid, 2'-Nitrobenzylidenacetessigsäuremethylester, 3-Nitrobenzylidenacetessigsäuredecylester, 2'-Trifluormethyl benzylidenacetessigsäureisopropylester, 2'-Cyanobenzylidenacetessigsäurecyclopentylester, 2'-Chlorbenzylidenacetessigsäure-(2-methoxyethyl)-ester, 2'-Methoxybenzylidenacetessigsäure-(2-cyanoethyl-ester, 2'-Methylhenzylidenacetessigsäurebenzylester, 3'-Nitrobenzylidenacetessigsäure-(pyridyl-2-methyl)-ester, 3'-Nitrobenzylidenaceffssigsäure-[2-(N-benzyl-N-methylamino)-ethyl]-ester, 3'-Nitro benzylidenacetessigsäure-(2-nitrooxyethyl) ester, α-Acetyl-β-(pyridyl-3)-acrylsäurepropylester, α-Acetyl-β-(pyridyl-2)-acrylsäureisobutylester, α-Acetyl-β-(chinolinyl-4-acrylsäure-(2-phenoxyethyl)-ester, α-Acetyl-β-(2.1.3-benzoxadiazolyl-4)-acrylsäuremethylester, α-Propionyl-β-(2.1.3-benzthiadiazolyl-4)-acrylsäureisopropylester.

In den Formeln II und III stehen

$R_4$, $R_5$, $R_6$ und $R_7$, die gleich oder verschieden sein können, vorzugsweise für Wasserstoff

oder

für einen geradkettigen oder verzweigten Alkylrest mit bis zu 8 Kohlenstoffatomen, der gegebenenfalls substituiert ist durch Hydroxy, Methoxy oder Acetoxy, wobei die Reste $R_4$ und $R_5$ sowie $R_6$ und $R_7$ mit dem jeweiligen Stickstoffatom einen 5 bis 7 gliedrigen Ring bilden können, der gegebenenfalls als weiteres Heteroatom Sauerstoff oder Schwefel oder eine N-Alkylgruppe mit bis zu 4 Kohlenstoffatomen oder eine N-Phenylgruppe enthält,

oder

vorzugsweise für einen Phenyl- oder Benzylrest.

Die als Ausgangsstoffe verwendeten 6-Aminopyrimidine der allgemeinen Formel III sind literaturbekannt oder können nach literaturbekannten Methoden hergestellt werden (vgl. z. B. Chem. Pharm. Bull. 19, 1526 (1971)).

Als Beispiele seien genannt :

2.4.6-Triamimopyrimidin, 6-Amino-2.4-bis-(diethylamino)-pyrimidin, 6-Amino-2.4-bis-(dibutylamino)-pyrimidin, 6-Amino-2.4-bis-(di-(2-methoxyethyl)-amino)-pyrimidin, 6-Amino-2.4-dimorpholino-pyrimidin, 6-Amino-2.4-dithiomorpholino-pyrimidin, 6-Amino-2.4-bis-(N-4-phenyl-piperazinyl- 1)-pyrimidin, 6-Amino-2.4-bis-(N-4-benzyl-piperazinyl-1)-pyrimidin.

Als Verdünnungsmittel kommen vorzugsweise organische Lösungsmittel in Frage, wie beispielweise Alkohole wie Methanol, Ethanol oder Isopropanol oder Ether wie Dioxan, Tetrahydrofuran, Glykolmonomethylether oder Glykoldimethylether oder Dimethylformamid, Dimethylsulfoxid, Acetonitril, Pyridin oder Hexamethylphosphorsäuretriamid oder Eisessig.

Die Reaktionstemperaturen können in einem größeren Bereich variiert werden. Im allgemeinen arbeitet man zwischen 20 °C und 150 °C, vorzugsweise zwischen 20 und 120 °C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol der Ylidenverbindung mit einem Mol 6-Aminopyrimidinderivat in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeigneten Trägermaterialien, unterwirft.

Verfahrensvariante B

Gemäß Verfahren B wird ein Aldehyd der allgemeinen Formel IV

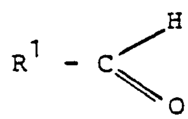

(IV)

mit einer β-Dicarbonylverbindung der allgemeinen Formel V

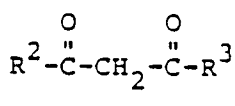

(V)

und einem 6-Aminopyrimidin der allgemeinen Formel III

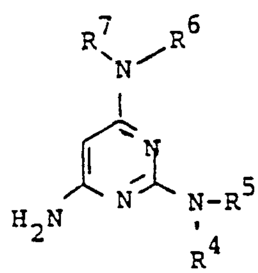

(III)

zur Reaktion gebracht.

In der Formel IV, V und III haben die Reste $R^1$, $R^2$, $R^3$, $R^4$, $R^5$, $R^6$ und $R^7$ die oben angegebene Bedeutung.

Die als Ausgangsstoffe verwendbaren Aldehyde der Formel IV sind literaturbekannt oder können nach literaturbekannten Methoden hergestellt werden (vgl. z. B. Mosettig, Org. Reactions VIII, 218 ff (1954)).

Als Beispiele seien genannt:

Benzaldehyd, 2-, 3- oder 4-Phenylbenzaldehyd, α- oder β-Naphthylaldehyd, 2-, 3- oder 4-Methylbenzaldehyd, 2- oder 4-n-Butylbenzaldehyd, 2-, 3- oder 4-Isopropylbenzaldehyd, 2- oder 4-Cyclopropylbenzaldehyd, 2,3-Tetramethylenbenzaldehyd, 3,4-Dioxymethylenbenzaldehyd, 2-, 3- oder 4-Methoxybenzaldehyd, 2-, 3- oder 4-Chlor/Brom/Fluorbenzaldehyd, 2-, 3- oder 4-Trifluormethylbenzaldehyd, 2-, 3- oder 4-Trifluormethoxybenzaldehyd, 2-, 3- oder 4-Difluormethoxybenzaldehyd, 2-, 3- oder 4-Nitrobenzaldehyd, 2-, 3- oder 4-Cyanobenzaldehyd, 3-Azidobenzaldehyd, 2-, 3- oder 4-Methylthiobenzaldehyd, 2-, 3- oder 4-Methylsulfinylbenzaldehyd, 2-, 3- oder 4-Methylsulfonylbenzaldehyd, 2,3-, 2,4- oder 2,6-Dichlorbenzaldehyd, 2-Fluor-3-chlorbenzaldehyd, 2.3.4.5.6-Pentafluorbenzaldehyd, 2,4-Dimethylbenzaldehyd, 2,4- oder 2,6-Dinitrobenzaldehyd, 2-Chlor-6-nitrobenzaldehyd, 4-Chlor-2-nitrobenzaldehyd, 2-Nitro-4-methoxybenzaldehyd, 2-Nitro-4-cyanobenzaldehyd, 2-Chlor-4-cyanobenzaldehyd, 4-Cyano-2-methylbenzaldehyd, 3-Methyl-4-trifluormethylbenzaldehyd, 3-Chlor-2-trifluormethylbenzaldehyd, Thiophen-2-aldehyd, Furan-2-aldehyd, Pyrrol-2-aldehyd, Pyrazol-4-aldehyd, Imidazol-2-aldehyd, Oxazol-2-aldehyd, Isoxazol-3-aldehyd, Thiazol-2-aldehyd, Pyridin-2-, 3- oder 4-aldehyd, 6-Methyl-pyridin-2-aldehyd, 2-Methylthio-pyridin-3-aldehyd, Indol-3-aldehyd, Benzimidazol-2-aldehyd, Benzoxazol-4-aldehyd, Benzoxadiazol-4-aldehyd, Chinolin-4-aldehyd, Chinazolin-2-aldehyd, Chinoxalin-5-aldehyd.

Die erfindungsgemäß verwendbaren β-Dicarbonylverbindungen der allgemeinen Formel V sind literaturbekannt oder können nach literaturbekannten Methoden hergestellt werden (z. B. D. Borrmann, « Umsetzung von Diketen mit Alkoholen, Phenolen und Mercaptanen », in Houben-Weyl, Methoden der Organischen Chemie, Vol. VII/4, 230 ff (1968) ; Y. Oikawa, K. Sugano und O. Yonemitsu, J. Org. Chem. 43, 2 087 (1978)).

Als Beispiele seien genannt :

Acetylaceton, Acetessigsäuremethylester, Acetessigsäuredecylester, Acetessigsäurecyclopentylester, Acetessigsäure-2,2,2-trifluorethylester, Acetessigsäure-(2-methoxyethyl)-ester, Acetessigsäure-(2-phenoxyethyl)-ester, Acetessigsäurebenzylester, Acetessigsäure-(2-(N-benzyl-N-methylamino)-ethyl)-ester, Acetessigsäure-(2-(pyridyl-3)-ethyl)-ester, Acetessigsäure-(2-acetoxyethyl)-ester, Acetessigsäure-(2-cyanoethyl)-ester, Acetessigsäure-(2-nitrooxyethyl)-ester, Acetessigsäureamid, Acetessigsäuredimethylamid, Acetessigsäureanilid, Propionylessigsäuremethylester, Benzoylessigsäureisobutylester.

Die als Ausgangsstoffe eingesetzten 6-Aminopyrimidine der allgemeinen Formel III wurden bereits unter Verfahrensvariante A angegeben.

Als Verdünnungsmittel kommen vorzugsweise organische Lösungsmittel in Frage, wie beispielsweise Alkohole wie Methanol, Ethanol oder Isopropanol oder Ether wie Dioxan, Tetrahydrofuran, Glykolmonomethylether oder Glykoldimethylether oder Dimethylformamid, Dimethylsulfoxid, Acetonitril, Pyridin, Hexamethylphosphorsäuretriamid oder Eisessig.

Die Reaktionstemperaturen können in einem größeren Bereich variiert werden. Im allgemeinen arbeitet man zwischen 20 °C und 150 °C, vorzugsweise zwischen 20 °C und 120 °C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol Aldehyd mit einem Mol β-Dicarbonylverbindung und einem Mol 6-Aminopyrimidin in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den erhaltenen Rückstand aus einem geeigneten Lösungsmittel umkristallisiert oder einer der aus dem Stand der Technik her bekannten Reinigungsmethoden, wie beispielsweise der Säulenchromatographie an geeigneten Trägermaterialien, unterwirft.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Je nach Wahl der Ausgangssubstanzen können die erfindungsgemäßen Verbindungen in stereoisomeren Formel, existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E.L. Eliel, Stereochemistry of Carbon Compounds, McGraw Hill, 1962).

Von besonderem Interesse sind Verbindungen der allgemeinen Formel (I), in welcher

$R^1$ für einen Phenyl- oder Naphthylrest oder für Thienyl, Furyl, Pyridyl, Pyridazinyl, Pyrimidyl, Benzoxadiazolyl, Benzthiadiazolyl, Chinolyl oder Isochinolyl steht, wobei die genannten Heterocyclen sowie der Phenyl- und der Naphthylrest 1 bis 5 gleiche oder verschiedene Substituenten tragen können, aus der Gruppe geradkettiges oder verzweigtes Alkyl mit bis zu 8 Kohlenstoffatomen, Alkoxyalkyl mit 1 bis 4 Kohlenstoffatomen, Dioxymethylen, Fluor, Chlor, Brom, Trifluormethyl, Trifluormethoxy, Difluormethoxy, Nitro, Cyano, Azido, Thioalkyl, Sulfonylalkyl mit jeweils 1 bis 4 C-Atomen im Alkylrest, Thiobenzyl und Sulfonylbenzyl,

$R^2$ für Alkyl mit bis zu 8 C-Atomen, Phenyl oder Benzyl steht,

oder

für den Rest $OR^8$ steht, wobei $R^8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Phenyl, Phenoxy, Phenylthio, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 C-Atomen, Phenyl oder Benzyl trägt,

$R^3$ für einen geradkettigen oder verzweigten Kohlenwasserstoffrest mit bis zu 6 C-Atomen steht, der gegebenenfalls substituiert ist durch 1 oder 2 Alkoxygruppen mit 1 bis 4 C-Atomen oder eine Acetoxygruppe,

oder

für einen Phenylrest oder Benzylrest steht, und

$R^4$, $R^5$, $R^6$ und $R^7$ jeweils gleich oder verschieden sind und für Wasserstoff oder einen geradkettigen oder verzweigten Alkylrest mit bis zu 8 Kohlenstoffatomen stehen, der gegebenenfalls substituiert ist durch Hydroxy, Methoxy oder Acetoxy und wobei die Reste $R^4$ und $R^5$ sowie $R^6$ und $R^7$ mit dem

jeweiligen Stickstoffatom einen 5 bis 7 gliedrigen Ring bilden können, der gegebenenfalls als weiteres Heteroatom Sauerstoff, Schwefel oder eine N-Alkylgruppe mit bis zu 4 C-Atomen oder eine N-phenylgruppe enthält.

Außer den unten angeführten Herstellungsbeispielen seien folgende erfindungsgemäßen Wirkstoffe genannt :

2.4-Diamino-7-propyl-5-(3-nitrophenyl)-5.8-dihydropyrido [2.3-d] pyrimidin-6-carbonsäureethylester,

2.4-Diamino-7-phenyl-5-(2-chlorphenyl)-5.8-dihydropyrido [2.3-d] pyrimidin-6-carbonsäuremethylester,

2.4-Diamino-7-benzyl-5-(2-trifluormethylphenyl)-5.8-dihydro-pyrido [2.3-d] pyrimidin-6-carbonsäuremethylester,

2.4-Diamino-7-methoxymethyl-5-(3-chlorphenyl)-5.8-dihydropyrido [2.3-d] pyrimidin-6-carbonsäureisopropylester,

2.4-Bis(dibutylamino)-7-methyl-5-(2.3-dichlorphenyl)-5.8-dihydro-pyrido [2.3-d] pyrimidin-6-carbonsäureethylester,

2.4-Bis(dibutylamino)-7-ethyl-5-(2-trifluormethylphenyl)-5.8-dihydro-pyrido [2.3-d] pyrimidin-6-carbonsäurecyclopentylester,

2.4-Bis(dibutylamino)-7-propyl-5-(3-nitrophenyl)-5.8-dihydro-pyrido [2.3-d] pyrimidin-6-carbonsäuredecylester,

2.4-Dipiperidino-7-methyl-5-(2-nitrophenyl)-5.8-dihydropyrido [2.3-d] pyrimidin-6-carbonsäureisobutylester,

2.4-Dipiperidino-7-phenyl-5-(2-chlor-3-trifluormethylphenyl)-5.8-dihydro-pyrido [2.3-d] pyrimidin-6-carbonsäure-(2-methoxyethyl)-ester,

2-Morpholino-4-piperidino-7-methyl-5-(2-cyanophenyl)-5.8-dihydro-pyrido [2.3-d] pyrimidin-6-carbonsäureisopropylester,

2-Morpholino-4-dimethylamino-7-methyl-5-(2.1.3-benzoxadiazolyl-4)-5.8-dihydro-pyrido [2.3-d] pyrimidin-6-carbonsäureethylester,

2.4-Dimorpholino-7-phenyl-5-(2.3-dichlorphenyl)-5.8-dihydro-pyrido [2.3-d] pyrimidin-6-carbonsäurebenzylester,

2.4-Dimorpholino-7-benzyl-5-(2-chlor-3-nitrophenyl)-5.8-dihydro-pyrido [2.3-d] pyrimidin-6-carbonsäureamid,

2.4-Dimorpholino-7-methyl-5-(3-trifluormethylphenyl)-5.8-dihydro-pyrido [2.3-d] pyrimidin-6-carbonsäuredimethylamid,

2.4-Dimorpholino-7-methyl-5-(pyridyl-3)-5.8-dihydropyrido [2.3-d] pyrimidin-6-carbonsäureanilid.

Die erfindungsgemäßen Verbindungen zeigen überraschenderweise im renalen Bereich besondere Wirkungen :

An narkotisierten Hunden unter künstlicher Beatmung üben die o.a. Verbindungen einen spezifisch renal gefäßdilatierenden Effekt aus, der sich in einer Erhöhung des Blutflusses in der A. renalis ausdrückt. Zur Feststellung des Effektes werden die Nierenarterien freigelegt und von umgebendem Gewebe befreit. Anschließend wird ein elektromagnetischer « Flow »-Meßkopf angebracht. Die selektive Flußerhöhung in den Nierenarterien wird nach Verabreichung von Dosen ab 0.03 mg/kg der erfindungsgemäßen Verbindungen beobachtet. Erst in höheren Dosen, etwa ab 1 mg/kg, werden Effekte auf die allgemeine Hämodynamik nachweisbar.

Darüberhinaus verfügen die erfindungsgemäßen Verbindungen über eine diuretische Wirkung, die an wachen Ratten nach oraler Gabe festzustellen ist. Hierzu werden wache Ratten mit 10 bis 30 ml Wasser bzw. 0,9 %iger Kochsalzlösung pro kg Körpergewicht belastet, mit Festsubstanz, die in Tylose-Suspension aufgenommen wird, oral behandelt und sodann zur Sammlung des Harnes über 6 Stunden in Stoffwechselkäfige gehalten. Die angegebenen Verbindungen erhöhen in Dosen ab 3 mg/kg p.o. die Harnvolumina, sowie die während dieses Zeitraumes insgesamt ausgeschiedenen Elektrolyte Natrium und Chlorid. Die renale Kaliumausscheidung ist im Vergleich zu unbehandelten Kontrolltieren nur gering erhöht.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen, vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum-, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie don oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Ethylalkohol, Propylenglykol, Suspendiermittel z. B. ethoxylierte Isostearylalkohole, Polyoxyethylensorbit- und sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung der oben angeführten Erkrankungen.

Die Wirkstoffe oder die pharmazeutischen Zubereitungen können oral, parenteral, intraperitoneal und/oder rektal, vorzugsweise oral, appliziert werden.

Im allgemeinen hat es sich als vorteilhaft erwiesen, den oder die Wirkstoffe bei i.v. Applikation in Mengen von etwa 0,01 bis etwa 20, vorzugsweise 0,1 bis 10,0 mg/kg Körpergewicht je 24 Stunden, verteilt auf 1 bis 6 Verabreichungen zu applizieren. Bei oraler Applikation werden vorzugsweise 0,05 bis 50 mg/kg, insbesondere 0,1 bis 20 mg/kg Körpergewicht gegeben, und zwar von oder/und während oder/und nach der Mahlzeit. Eine Einzelgabe enthält den oder die Wirkstoffe, vorzugsweise in Mengen von etwa 0,01 bis etwa 10,0 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verarbreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

2.4-Diamino-7-methyl-5-phenyl-5.8-dihydro-pyrido [2.3-d] pyrimidin-6-carbonsäureethylester

(Siehe Formel Seite 10 f.)

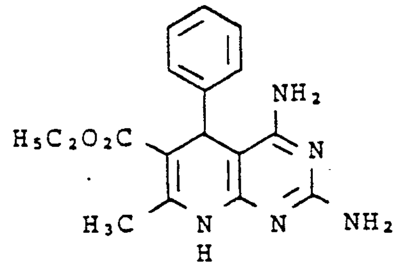

2.2 g (10 mMol) 2-Benzylidenacetessigsäureethylester wurden zusammen mit 1.3 g (10 mMol) 2.4.6-Triaminopyrimidin in 25 ml Eisessig 10 Stunden unter Rückfluß erhitzt. Anschließend wurde die Reaktionsmischung auf Zimmertemperatur abgekühlt, das ausgefallene Produkt abgesaugt und aus Acetonitril als Acetat umkristallisiert. Schmelzpunkt : 145 °C Ausbeute : 2.8 g (73 %).

Beispiel 2

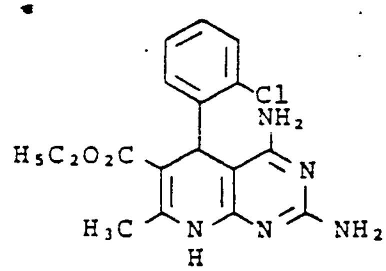

Analog Beispiel 1 wurde durch Umsetzung von 2-(2-Chlorbenzyliden)-acetessigsäureethylester mit 2.4.6-Triaminopyrimidin der 2.4-Diamino-7-methyl-5-(2-chlorphenyl)-5.8-dihydro-pyrido [2.3-d] pyrimidin-6-carbonsäureethylester als Acetat erhalten, Fp : 150 °C (Acetonitril). Ausbeute : 60 %.

Beispiel 3

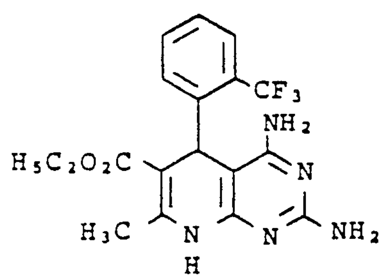

Analog Beispiel 1 wurde durch Umsetzung von 2-(2-Trifluormethylbenzyliden)-acetessigsäureethylester mit 2.4.6-Triaminopyrimidin der 2.4-Diamino-7-methyl-5-(2-trifluormethylphenyl)-5.8-dihydro-pyrido [2.3-d] pyrimidin-6-carbonsäureethylester als Acetat erhalten, Fp.: 160 °C (Isopropanol). Ausbeute : 58 %.

Beispiel 4

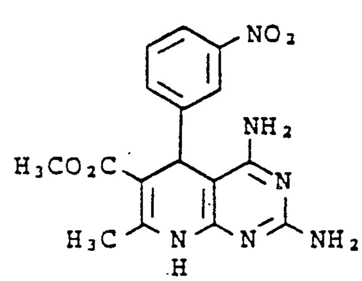

Analog Beispiel 1 wurde durch Umsetzung von 2-(3-Nitrobenzyliden)-acetessigsäuremethylester mit 2.4.6-Triaminopyrimidin der 2.4-Diamino-7-methyl-5-(3-nitrophenyl)-5.8-dihydro-pyrido[2.3-d]pyrimidin-6-carbonsäuremethylester als Acetat erhalten, Fp. : 250 °C (Acetonitril). Ausbeute : 73 %.

Beispiel 5

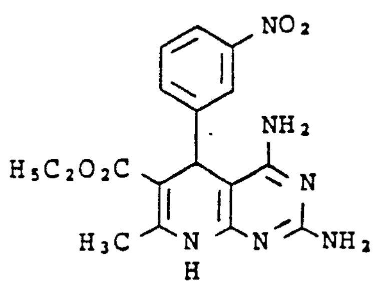

Analog Beispiel 1 wurde durch Umsetzung von 2-(3-Nitrobenzyliden)-acetessigsäureethylester mit 2.4.6-Triaminopyrimidin der 2.4-Diamino-7-methyl-5-(3-nitrophenyl)-5.8-dihydro-pyrido[2.3-d]pyrimidin-6-carbonsäureethylester vom Fp. : 230 °C (Ethanol) erhalten. Ausbeute : 78 %.

Beispiel 6

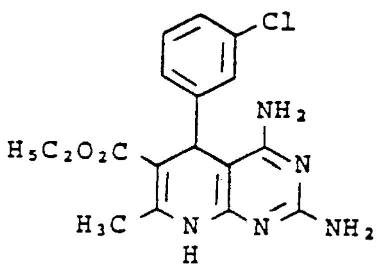

Analog Beispiel 1 wurde durch Umsetzung von 2-(3-Chlorbenzyliden)-acetessigsäureethylester mit 2.4.6-Triaminopyrimidin der 2.4-Diamino-7-methyl-5-(3-chlorphenyl)-5.8-dihydro-pyrido[2.3-d]pyrimidin-6-carbonsäureethylester als Acetat erhalten, Fp. : 136 °C (Acetonitril). Ausbeute : 59 %.

Beispiel 7

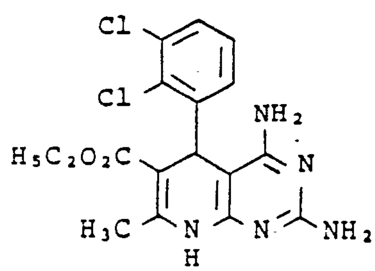

Analog Beispiel 1 wurde durch Umsetzung von 2-(2.3-Dichlorbenzyliden)-acetessigsäureethylester mit 2.4.6-Triaminopyrimidin der 2.4-Diamino-7-methyl-5-(2.3-dichlorphenyl)-5.8-dihydro-pyrido[2.3-d]pyrimidin-6-carbonsäureethylester als Acetat erhalten, Fp. : 177 °C (Ethanol). Ausbeute : 70 %.

Beispiel 8

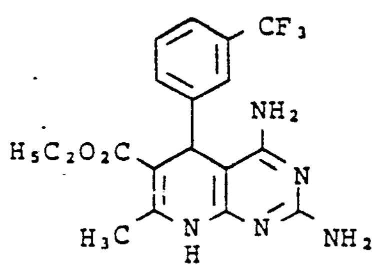

Analog Beispiel 1 wurde durch Umsetzung von 2-(3-Trifluormethylbenzyliden)-acetessigsäureethylester mit 2.4.6.-Triaminopyrimidin in Eisessig der 2.4-Diamino-7-methyl-5-(3-trifluormethylphenyl)-5.8-dihydro-pyrido[2.3-d]pyrimidin-6-carbonsäureethylester als Diacetat vom Fp.: 123 °C. (Ethanol) erhalten. Ausbeute : 61 %.

Beispiel 9

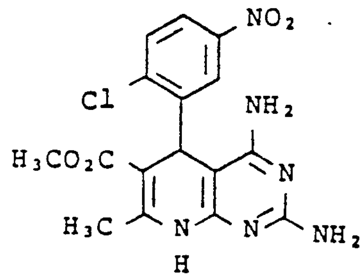

Analog Beispiel 1 wurde durch Umsetzung von 2-(2-Chlor-5-nitro-benzyliden)-acetessigsäuremethylester mit 2.4.6-Triaminopyrimidin in Eisessig der 2.4-Diamino-7-methyl-5-(2-chlor-5-nitrophenyl)-5.8-dihydro-pyrido[2.3-d]pyrimidin-6-carbonsäuremethylester als Acetat von Fp.: 282 °C (Ethanol) erhalten. Ausbeute : 55 %.

Beispiel 10

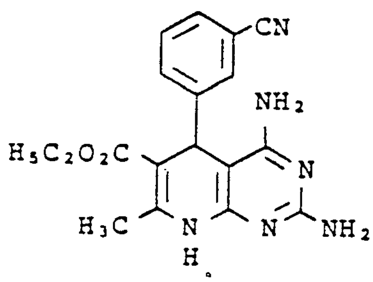

Analog Beispiel 1 wurde durch Umsetzung von 2-(3-Cyanobenzyliden)-acetessigsäureethylester mit 2.4.6-Triaminopyrimidin in Eisessig der 2.4-Diamino-7-methyl-5-(3-cyanophenyl)-5.8-dihydropyrido[2.3-d]pyrimidin-6-carbonsäureethylester als Acetat vom Fp. : 170 °C (Acetonitril) erhalten. Ausbeute : 61 %.

Beispiel 11

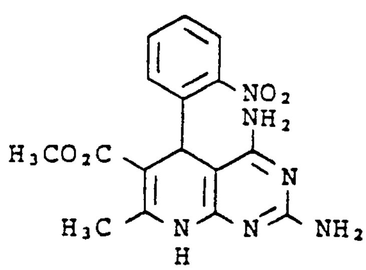

Analog Beispiel 1 wurde durch Umsetzung von 2-(2-Nitrobenzyliden)-acetessigsäuremethylester mit 2.4.6-Triaminopyrimidin in Eisessig der 2.4-Diamino-7-methyl-5-(2-nitrophenyl)-5.8-dihydro-pyrido[2.3-d]pyrimidin-6-carbonsäuremethylester als Acetat erhalten, Fp. : 164 °C (Ethanol). Ausbeute : 65 %.

Beispiel 12

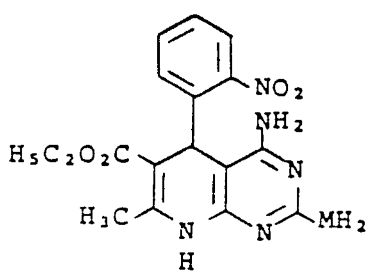

Analog Beispiel 1 wurde durch Umsetzung von 2-(2-Nitrobenzyliden)-acetessigsäureethylester mit 2.4.6-Triaminopyrimidin in Eisessig der 2.4-Diamino-7-methyl-5-(2-nitrophenyl)-5.8-dihydro-pyrido[2.3-d]pyrimidin-6-carbonsäureethylester als Acetat erhalten, Fp. : 196 °C (Ethanol). Ausbeute : 67 %.

Beispiel 13

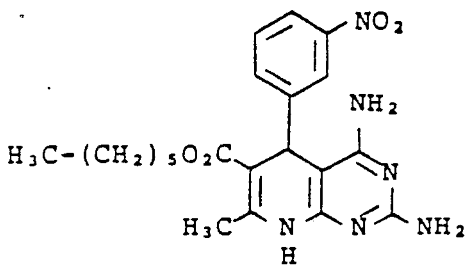

Analog Beispiel 1 wurde durch Umsetzung von 2-(3-Nitrobenzyliden)-acetessigsäurehexylester mit 2.4.6-Triaminopyrimidin in Eisessig der 2.4-Diamino-7-methyl-5-(3-nitrophenyl)-5.8-dihydro-pyrido[2.3-d]pyrimidin-6-carbonsäurehexylester als Diacetat erhalten, Fp. : 132 °C (Essigester). Ausbeute : 59 %.

Beispiel 14

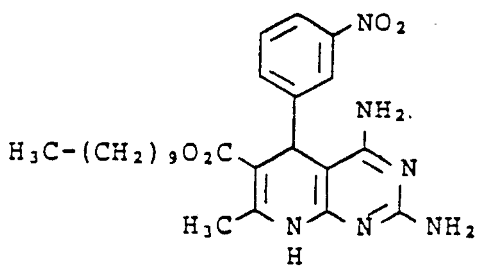

Analog Beispiel 1 wurde durch Umsetzung von 2-(3-Nitrobenzyliden)-acetessigsäuredecylester mit 2.4.6-Triaminopyrimidin in Eisessig der 2.4-Diamino-7-methyl-5-(3-nitrophenyl)-5.8-dihydro-pyrido[2.3-d]pyrimidin-6-carbonsäuredecylester als Acetat vom Fp. : 103 °C (Essigester) erhalten. Ausbeute : 56 %.

Beispiel 15

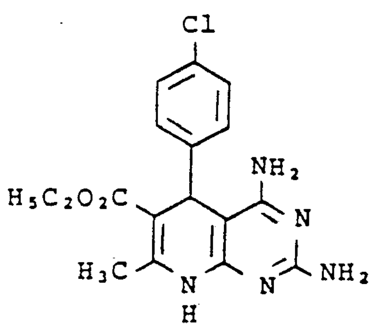

Analog Beispiel 1 wurde durch Umsetzung von 2-(4-Chlorbenzyliden)-acetessigsäureethylester mit 2.4.6-Triaminopyrimidin in Eisessig der 2.4-Diamino-7-methyl-5-(4-chlorphenyl)-5.8-dihydro-pyrido[2.3-d]pyrimidin-6-carbonsäureethylester als Halbacetat erhalten, Fp. : 128 °C (Isopropanol). Ausbeute 75 %.

Beispiel 16

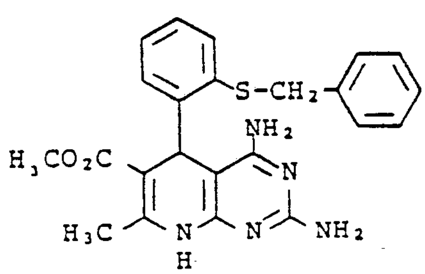

Analog Beispiel 1 wurde durch Umsetzung von 2-(2-Benzylthiophenyl)-acetessigsäuremethylester mit 2.4.6-Triaminopyrimidin in Eisessig der 2.4-Diamino-7-methyl-5-(2-benzylthiophenyl)-5.8-dihydro-pyrido[2.3-d]pyrimidin-6-carbonsäuremethylester als Acetat vom Fp. : 139 °C (Methanol) erhalten. Ausbeute : 50 %.

Beispiel 17

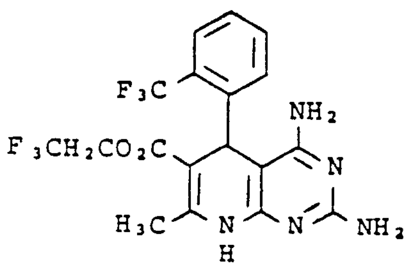

Analog Beispiel 1 wurde durch Umsetzung von 2-(2-Trifluormethylbenzyliden)-acetessigsäure-(2.2.2-trifluorethyl)ester mit 2.4.6-Triaminopyrimidin in Eisessig der 2.4-Diamino-7-methyl-5-(2-trifluormethylphenyl)-5.8-dihydropyrido[2.3-d]pyrimidin-6-carbonsäure-(2.2.2-trifluorehyl)-ester als Acetat erhalten, Fp. : 145 °C (Ethanol). Ausbeute : 52 %.

Beispiel 18

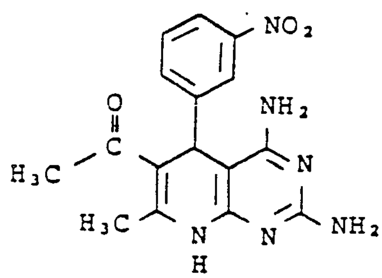

Analog Beispiel 1 wurde durch Umsetzung von 2-(3-Nitrobenzyliden)-acetylaceton mit 2.4.6-Triaminopyrimidin in Eisessig das 6-Acetyl-2.4-diamino-7-methyl-5-(3-nitrophenyl)-5.8-dihydro-pyrido[2.3-d]pyrimidin als Acetat vom Fp. : 260 °C (Ethanol) erhalten. Ausbeute : 73 %.

Beispiel 19

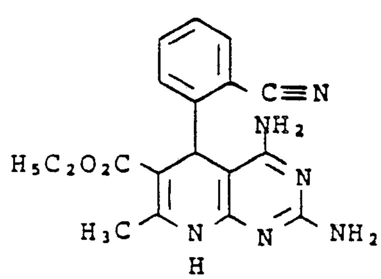

Analog Beispiel 1 wurde durch Umsetzung von 2-(2-Cyanobenzyliden)-acetessigsäureethylester mit 2.4.6-Triaminopyrimidin in Eisessig der 2.4-Diamino-7-methyl-5-(2-cyanophenyl)-5.8-dihydro-pyrido[2.3-d]pyrimidin-6-carbonsäureethylester als Acetat erhalten, Fp. : 208 °C (Ethanol). Ausbeute : 59 %.

(Siehe Formel Seite 15 f.)

Beispiel 20

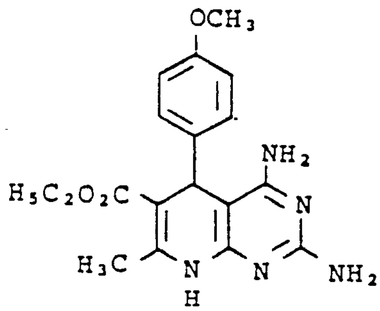

Analog Beispiel 1 wurde durch Umsetzung von 2-(4-Methoxybenzyliden)-acetessigsäureethylester mit 2.4.6-Triaminopyrimidin in Eisessig der 2.4-Diamino-7-methyl-5-(4-methoxyphenyl)-5.8-dihydro-pyrido[2.3-d]pyrimidin-6-carbonsäureethylester vom Fp. : 98 °C (Essigester) erhalten. Ausbeute : 75 %.

Beispiel 21

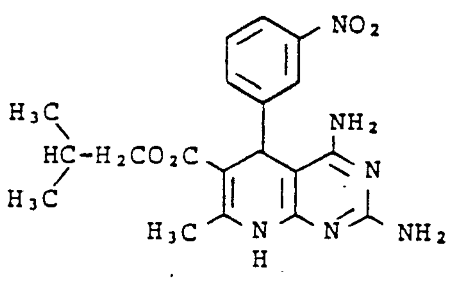

Analog Beispiel 1 wurde durch Umsetzung von 2-(3-Nitrobenzyliden)-acetessigsäureisobutylester mit 2.4.6-Triaminopyrimidin in Eisessig der 2.4-Diamino-7-methyl-5-(3-nitrophenyl)-5.8-dihydro-pyrido[2.3-d]pyrimidin-6-carbonsäureisobutylester vom Fp. : 130 °C erhalten. Ausbeute : 78 %.

Beispiel 22

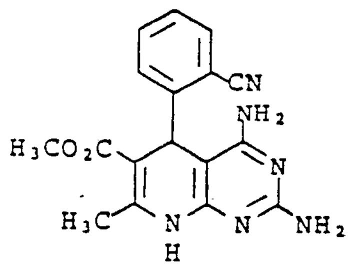

Analog Beispiel 1 wurde durch Umsetzung von 2-(2-Cyanobenzyliden)-acetessigsäuremethylester mit 2.4.6-Triaminopyrimidin in Eisessig der 2.4-Diamino-7-methyl-5-(2-cyanophenyl)-5.8-dihydro-pyrido[2.3-d]pyrimidin-6-carbonsäuremethylester als Halbacetat erhalten, Fp. : 170 °C (Methanol). Ausbeute : 55 %.

Beispiel 23

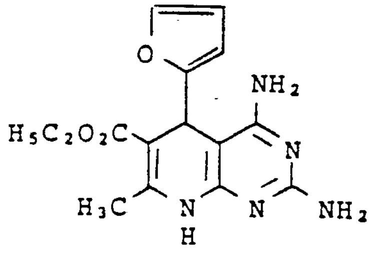

Analog Beispiel 1 wurde durch Umsetzung von 2-Acetyl-3-(2-furyl)-acrylsäureethylester mit 2.4.6-Triaminopyrimidin in Eisessig der 2.4-Diamino-7-methyl-5-(furyl-2)-5.8-dihydropyrido [2.3-d]pyrimidin-6-carbonsäureethylester als Acetat vom Fp. : 106 °C (Acetonitril) erhalten. Ausbeute : 57 %.

Beispiel 24

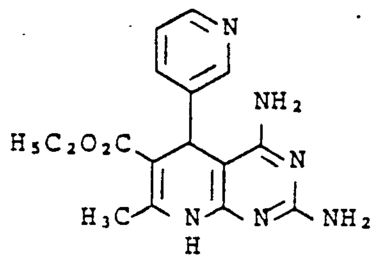

Analog Beispiel 1 wurde durch Umsetzung von 2-Acetyl-3-(pyridyl-3)-acrylsäureethylester mit 2.4.6-Triaminopyrimidin in Eisessig der 2.4-Diamino-7-methyl-5-(pyridyl-3)-5.8-dihydro-pyrido[2.3-d]pyrimidin-6-carbonsäureethylester vom Fp. : 224 °C (Methanol) erhalten. Ausbeute : 63 %.

Beispiel 25

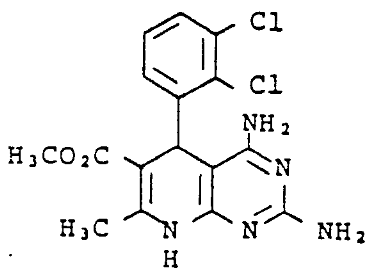

Analog Beispiel 1 wurde durch Umsetzung von 2-(2.3-Dichlorbenzyliden)-acetessigsäureethylester mit 2.4.6-Triaminopyrimidin in Eisessig der 2.4-Diamino-7-methyl-5-(2.3-dichlorphenyl)-5.8-dihydro-pyrido[2.3-d]pyrimidin-6-carbonsäuremethylester als Acetat vom Fp. : 160 °C (Ethanol) erhalten. Ausbeute : 72 %.

Beispiel 26

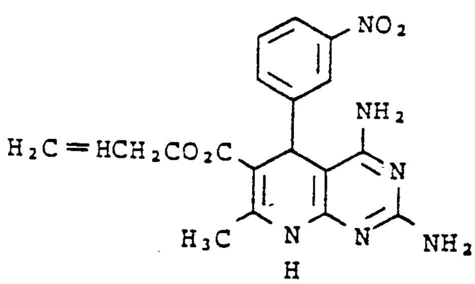

Analog Beispiel 1 wurde durch Umsetzung von 2-(3-Nitrobenzyliden)-acetessigsäureallylester mit 2.4.6-Triaminopyrimidin in Eisessig der 2.4-Diamino-7-methyl-5-(3-nitrophenyl)-5.8-dihydro-pyrido[2.3-d]pyrimidin-6-carbonsäureallylester vom Fp. : 188 °C (Essigester) erhalten. Ausbeute : 64 %.

Beispiel 27

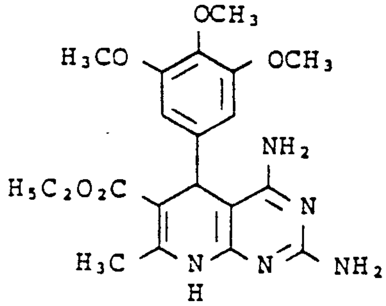

Analog Beispiel 1 wurde durch Umsetzung von 2-(3.4.5-Trimethoxybenzyliden)-acetessigsäureethylester mit 2.4.6-Triaminopyrimidin in Eisessig der 2.4-Diamino-7-methyl-5-(3.4.5-trimethoxyphenyl)-5.8-dihydro-pyrido[2.3-d]pyrimidin-6-carbonsäureethylester vom Fp. : 126 °C (Essigester) erhalten. Ausbeute : 69 %.

Beispiel 28

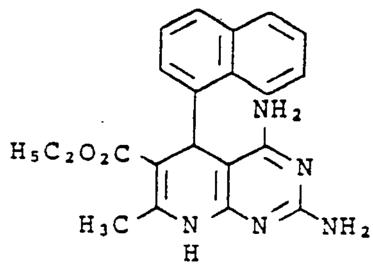

Analog Beispiel 1 wurde durch Umsetzung von 2-Acetyl-3-(naphthyl-1)-acrylsäureethylester mit 2.4.6-Triaminopyrimidin in Eisessig der 2.4-Diamino-7-methyl-5-(naphthyl-1)-5.8-dihydro-pyrido[2.3-d]pyrimidin-6-carbonsäureethylester als Halbacetat vom Fp. : 209 °C (Methanol) erhalten. Ausbeute : 35 %.

Beispiel 29

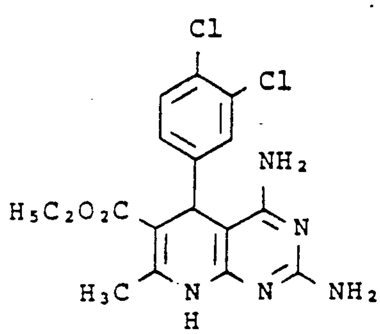

Analog Beispiel 1 wurde durch Umsetzung von 2-(3.4-Dichlorbenzyliden)-acetessigsäureethylester mit 2.4.6-Triaminopyrimidin in Eisessig der 2.4-Diamino-7-methyl-5-(3.4-dichlorphenyl)-5.8-dihydro-pyrido[2.3-d]pyrimidin-6-carbonsäureethylester vom Fp. : 244 °C (Ethanol) erhalten. Ausbeute : 76 %.

Beispiel 30

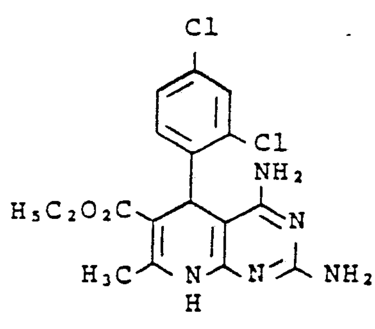

Analog Beispiel 1 wurde durch Umsetzung von 2-(2.4-Dichlorbenzyliden)-acetessigsäureethylester mit 2.4.6-Triaminopyrimidin in Eisessig der 2.4-Diamino-7-methyl-5-(2.4-dichlorphenyl)-5.8-dihydro-pyrido[2.3-d]pyrimidin-6-carbonsäureethylester als Diacetat vom Fp. : 148 °C (Ethanol) erhalten. Ausbeute : 72 %.

(Siehe Formel Seite 18 f.)

Beispiel 31

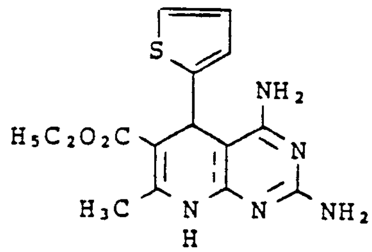

Analog Beispiel 1 wurde durch Umsetzung von 2-Acetyl-3-(thienyl-2)-acrylsäureethylester mit 2.4.6-Triaminopyrimidin in Eisessig der 2.4-Diamino-7-methyl-5-(thienyl-2)-5.8-dihydropyrido[2.3-d]pyrimidin-6-carbonsäureethylester als Diacetat erhalten, Fp. : 126 °C (Methanol). Ausbeute : 51 %.

Beispiel 32

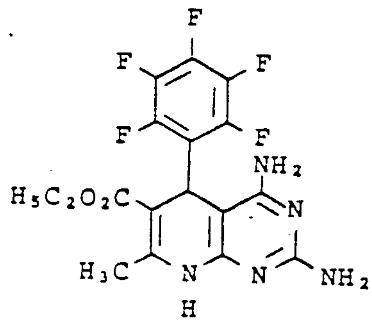

Analog Beispiel 1 wurde durch Umsetzung von 2-(2.3.4.5.6-Pentafluorbenzyliden)-acetessigsäureethylester mit 2.4.6-Triaminopyrimidin in Eisessig der 2.4-Diamino-7-methyl-5-(pentafluorphenyl)-5.8-dihydro-pyrido[2.3-d]pyrimidin-6-carbonsäureethylester als Triacetat vom Fp. : 162 °C (Essigsäure) erhalten. Ausbeute : 37 %.

Beispiel 33

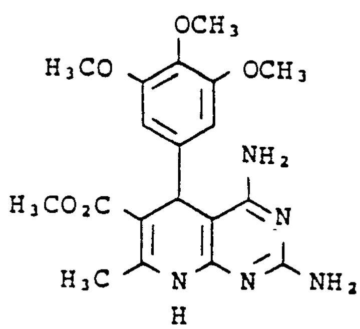

Analog Beispiel 1 wurde durch Umsetzung von 2-(3.4.5-Trimethoxybenzyliden)-acetessigsäuremethylester mit 2.4.6-Triaminopyrimidin in Eisessig der 2.4-Diamino-7-methyl-5-(3.4.5-trimethoxyphenyl)-5.8-dihydro-pyrido[2.3-d]pyrimidin-6-carbonsäuremethylester als Acetat vom Fp. : 168 °C (Wasser) erhalten. Ausbeute : 72 %.

Beispiel 34

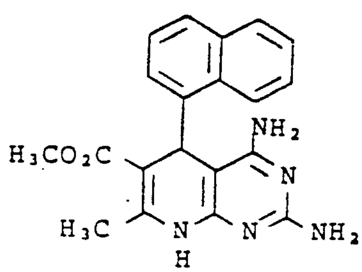

Analog Beispiel 1 wurde durch Umsetzung von 2-Acetyl-3-(naphthyl-1)-acrylsäuremethylester mit 2.4.6-Triaminopyrimidin in Eisessig der 2.4-Diamino-7-methyl-5-(naphthyl-1)-5.8-dihydro-pyrido[2.3-d]pyrimidin-6-carbonsäuremethylester als Acetat vom Fp. : 210 °C (Essigester/Methanol) erhalten. Ausbeute : 36 %.

Beispiel 35

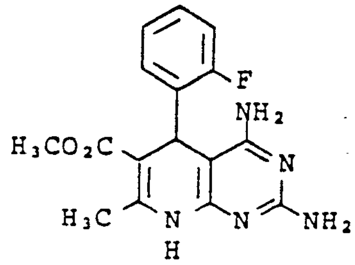

Analog Beispiel 1 wurde durch Umsetzung von 2-(2-Fluorbenzyliden)-acetessigsäuremethylester mit 2.4.6-Triaminopyrimidin in Eisessig der 2.4-Diamino-7-methyl-5-(2-fluorphenyl)-5.8-dihydro-pyrido[2.3-d]pyrimidin-6-carbonsäuremethylester vom Fp. : 208 °C (Isopropanol) erhalten. Ausbeute : 76 %.

Beispiel 36

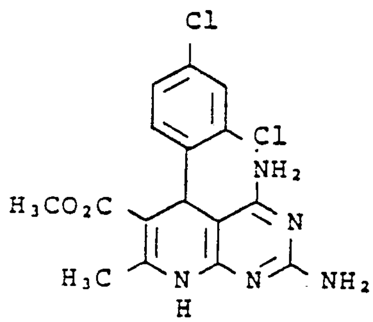

Analog Beispiel 1 wurde durch Umsetzung von 2-(2.4-Dichlorbenzyliden)-acetessigsäuremethylester mit 2.4.6-Triaminopyrimidin in Eisessig der 2.4-Diamino-7-methyl-5-(2.4-dichlorphenyl)-5.8-dihydro-pyrido[2.3-d]pyrimidin-6-carbonsäuremethylester als Acetat erhalten, Fp. : 150 °C (Ethanol). Ausbeute : 71 %.

Beispiel 37

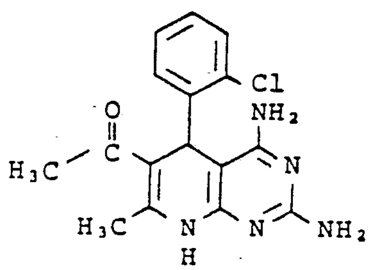

Analog Beispiel 1 wurde durch Umsetzung von 2-(2-Chlorbenzyliden)-acetylaceton mit 2.4.6-Triaminopyrimidin in Eisessig das 6-Acetyl-2.4-diamino-7-methyl-5-(2-chlorphenyl)-5.8-dihydro-pyrido[2.3-d]pyrimidin vom Fp. : 254 °C (Essigester) erhalten. Ausbeute : 70 %.

(Siehe Formel Seite 20 f.)

Beispiel 38

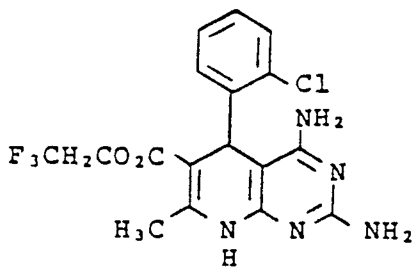

Analog Beispiel 1 wurde durch Umsetzung von 2-(2-Chlorbenzyliden)-acetessigsäure-(2.2.2-trifluorethyl)-ester mit 2.4.6-Triaminopyrimidin in Eisessig der 2.4-Diamino-7-methyl-5-(2-chlorphenyl)-5.8-dihydro-pyrido[2.3-d]pyrimidin-6-carbonsäure-(2.2.2-trifluorethyl)-ester als Diacetat vom Fp. : 146 °C erhalten. Ausbeute : 53 %.

Beispiel 39

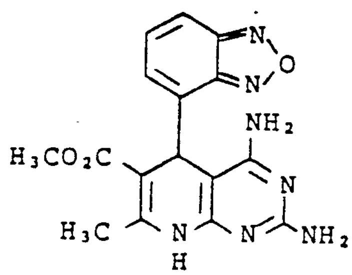

Analog Beispiel 1 wurde durch Umsetzung von 2-Acetyl-3-(2.1.3-benzoxadiazolyl-4)-acrylsäuremethylester mit 2.4.6-Triaminopyrimidin in Eisessig der 2.4-Diamino-7-methyl-5-(2.1.3-benzoxadiazolyl-4)-5.8-dihydro-pyrido[2.3-d]pyrimidin-6-carbonsäuremethylester vom Fp. : 268 °C (Ethanol) erhalten. Ausbeute : 51 %.

Beispiel 40

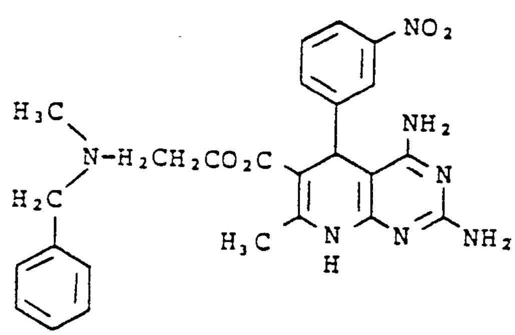

Analog Beispiel 1 wurde durch Umsetzung von 2-(3-Nitrobenzyliden)-acetessigsäure-(2-(N-benzyl-N-methylamino)-ethyl)-ester mit 2.4.6-Triaminopyrimidin in Eisessig der 2.4-Diamino-7-methyl-5-(3-nitrophenyl)-5.8-dihydropyrido[2.3-d]pyrimidin-6-carbonsäure-(2-(N-benzyl-N-methylamino)-ethyl)-ester vom Fp. : 162 °C (Essigester) erhalten. Ausbeute : 53 %.

Beispiel 41

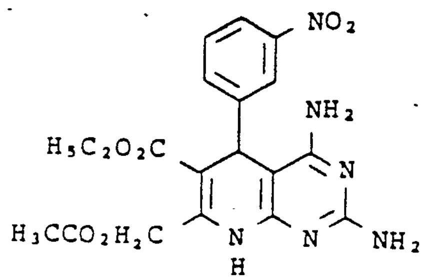

Analog Beispiel 1 wurde durch Umsetzung von 4-Acetoxy-2-(3-nitrobenzyliden)-acetessigsäureethylester mit 2.4.6-Triaminopyrimidin in Eisessig der 7-Acetoxymethyl-2.4-diamino-5-(3-nitrophenyl)-5.8-dihydro-pyrido[2.3-d]pyrimidin-6-carbonsäureethylester vom Fp. : 219 °C erhalten. Ausbeute : 65 %.

Beispiel 42

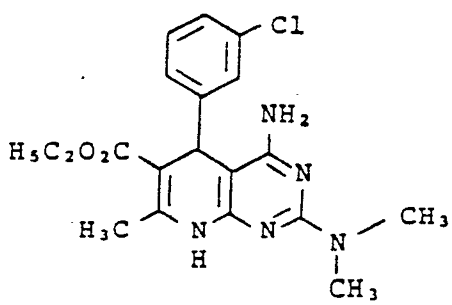

Analog Beispiel 1 wurde durch Umsetzung von 2-(3-Chlorbenzyliden)-acetessigsäureethylester mit 2-Dimethylamino-4.6-diamino-pyrimidin in Eisessig der 4-Amino-2-dimethylamino-7-methyl-5-(3-chlorphenyl)-5.8-dihydro-pyrido[2.3-d]pyrimidin-6-carbonsäureethylester vom Fp. : 214 °C (Ethanol) erhalten. Ausbeute : 55 %.

Beispiel 43

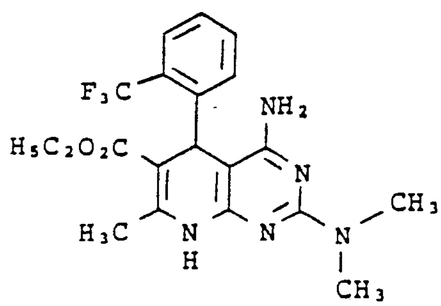

Analog Beispiel 1 wurde durch Umsetzung von 2-(2-Trifluormethylbenzyliden)-acetessigsäureethylester mit 2-Dimethylamino-4.6-diamino-pyrimidin in Eisessig der 4-Amino-2-dimethylamino-7-methyl-5-(2-trifluormethylphenyl)-5.8-dihydro-pyrido[2.3-d]pyrimidin-6-carbonsäureethylester vom Fp. : 210 °C (Ethanol) erhalten. Ausbeute : 61 %.

Beispiel 44

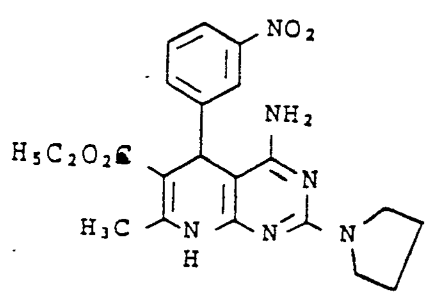

Analog Beispiel 1 wurde durch Umsetzung von 2-(3-Nitrobenzyliden)-acetessigsäureethylester mit 2-Pyrrolidino-4.6-diamino-pyrimidin in Eisessig der 4-Amino-2-pyrrolidino-7-methyl-5-(3-nitrophenyl)-5.8-dihydro[2.3-d]pyrimidin-6-carbonsäureethylester als Diacetat erhalten, Fp. : 243 °C (Isopropanol). Ausbeute : 59 %.

(Siehe Formel Seite 22 f.)

Beispiel 45

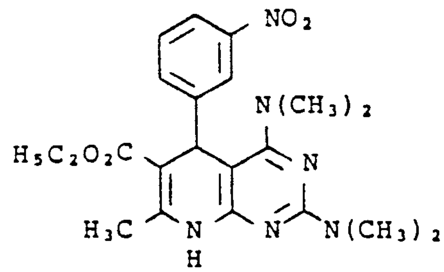

Analog Beispiel 1 wurde durch Umsetzung von 2-(3-Nitrobenzyliden)-acetessigsäureethylester mit 6-Amino-2.4-bis-(dimethylamino)-pyrimidin in Eisessig der 2.4-Bis-(dimethylamino)-7-methyl-5-(3-nitrophenyl)-5.8-dihydropyrido[2.3-d]pyrimidin-6-carbonsäureethylester vom Fp. : 149 °C (Isopropanol) erhalten. Ausbeute : 55 %.

Beispiel 46

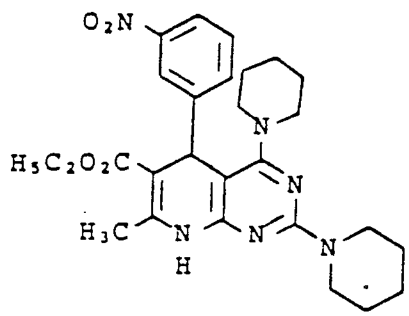

Analog Beispiel 1 wurde durch Umsetzung von 2-(3-Nitrobenzyliden)-acetessigsäureethylester mit 6-Amino-2.4-dipiperidino-pyrimidin in Eisessig der 2.4-Dipiperidino-7-methyl-5-(3-nitrophenyl)-5.8-dihydro-pyrido[2.3-d]pyrimidin-6-carbonsäureethylester vom Fp. : 169 °C (Ethanol/Wasser) erhalten. Ausbeute : 45 %.

Beispiel 47

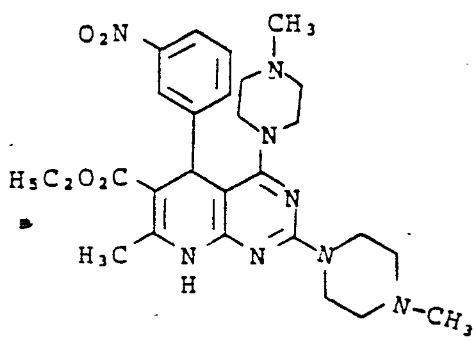

Analog Beispiel 1 wurde durch Umsetzung von 2-(3-Nitrobenzyliden)-acetessigsäureethylester mit 6-Amino-2.4-bis-(N-4-methylpiperazinyl-1)-pyrimidin in Eisessig der 2.4-Bis-(N-4-methylpiperazinyl-1)-7-methyl-5-(3-nitrophenyl)-5.8-dihydro-pyrido[2.3-d]pyrimidin-6-carbonsäureethylester vom Fp. : 140 °C (Methanol) erhalten. Ausbeute : 43 %.

(Siehe Formel Seite 23 f.)

Beispiel 48

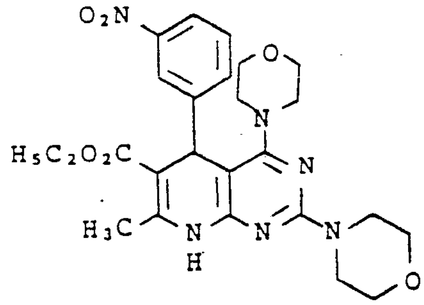

Analog Beispiel 1 wurde durch Umsetzung von 2-(3-Nitrobenzyliden)-acetessigsäureethylester mit 6-Amino-2.4-dimorpholino-pyrimidin in Eisessig der 2.4-Dimorpholino7-methyl-5-(3-nitrophenyl)-5.8-dihydro-pyrido[2.3-d]-pyrimidin-6-carbonsäureethylester vom Fp.: 190 °C (Ethanol) erhalten. Ausbeute: 67 %.

Beispiel 49

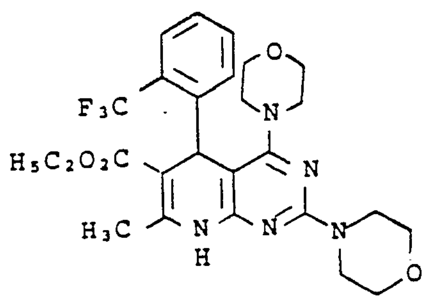

Analog Beispiel 1 wurde durch Umsetzung von 2-(2-Trifluormethylbenzyliden)-acetessigsäureethylester mit 6-Amino-2.4-dimorpholino-pyrimidin in Eisessig der 2.4-Dimorpholino-7-methyl-5-(2-trifluormethylphenyl)-5.8-dihydro-pyrido[2.3-d]pyrimidin-6-carbonsäureethylester vom Fp.: 214 °C (Isopropanol) erhalten. Ausbeute: 66 %.

Beispiel 50

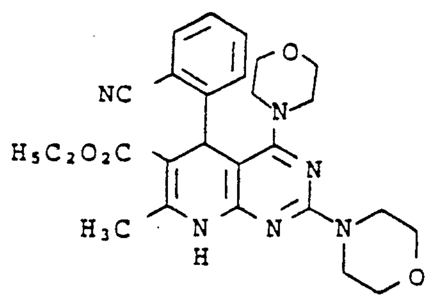

Analog Beispiel 1 wurde durch Umsetzung von 2-(2-Cyanobenzyliden)-acetessigsäureethylester mit 6-Amino-2.4-dimorpholino-pyrimidin in Ethanol der 2.4-Dimorpholino7-methyl-5-(2-cyanophenyl)-5.8-dihydro-pyrido[2.3-d]pyrimidin-6-carbonsäureethylester vom Fp.: 248 °C (Isopropanol) erhalten. Ausbeute: 75 %.

Beispiel 51

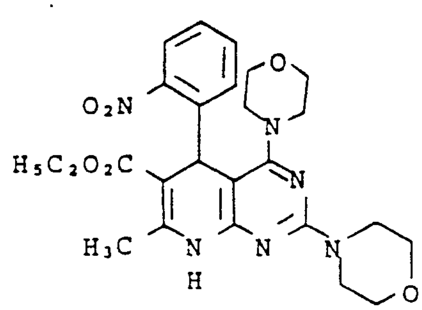

Analog Beispiel 1 wurde durch Umsetzung von 2-(2-Nitrobenzyliden)-acetessigsäureethylester mit 6-Amino-2.4-dimorpholino-pyrimidin in Ethanol der 2.4-Dimorpholino-7-methyl-5-(2-nitrophenyl)-5.8-dihydro-pyrido[2.3-d]pyrimidin-6-carbonsäureethylester vom Fp. : 201 °C (Isopropanol) erhalten. Ausbeute : 65 %.

Beispiel 52

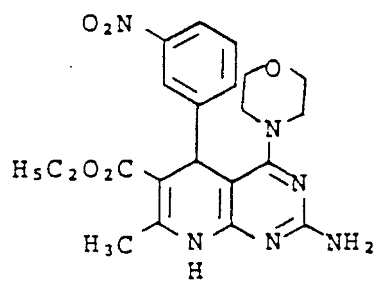

Analog Beispiel 1 wurde durch Umsetzung von 2-(3-Nitrobenzyliden)-acetessigsäureethylester mit 2.6-Diamino-4-morpholino-pyrimidin in Ethanol der 2-Amino-4-morpholino-7-methyl-5-(3-nitrophenyl)-5.8-dihydro-pyrido[2.3-d]pyrimidin-6-carbonsäureethylester vom Fp. : 208 °C (Ethanol) erhalten. Ausbeute : 79 %.

Beispiel 53

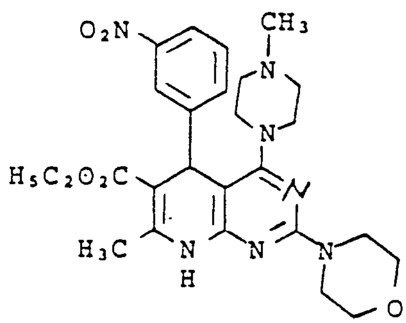

Analog Beispiel 1 wurde durch Umsetzung von 2-(3-Nitrobenzyliden)-acetessigsäureethylester mit 6-Amino-2-morpholino-4-(N-4-methylpiperazinyl-1)-pyrimidin in Ethanol der 2-Morpholino-4-(N-4-methylpiperazinyl-1)-7-methyl-5-(3-nitrophenyl)-5.8-dihydro-pyrido[2.3-d]pyrimidin-6-carbonsäureethylester vom Fp. : 218 °C (Ethanol) erhalten. Ausbeute : 77 %.

Beispiel 54

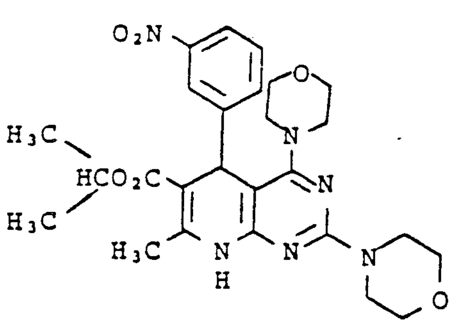

Analog Beispiel 1 wurde durch Umsetzung von 2-(3-Nitrobenzyliden)-acetessigsäureisopropylester mit 6-Amino-2.4-dimorpholino-pyrimidin in Ethanol der 2.4-Dimorpholino-7-methyl-5-(3-nitrophenyl)-5.8-dihydro-pyrido[2.3-d]pyrimidin-6-carbonsäureisopropyleshter vom Fp. : 229 °C (Ethanol). Ausbeute : 84 %.

Beispiel 55

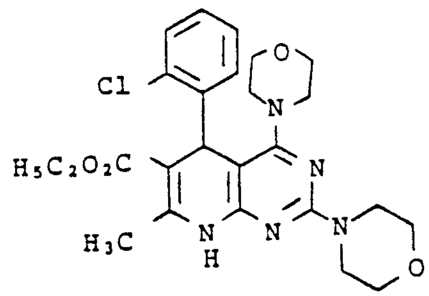

Analog Beispiel 1 wurde durch Umsetzung von 2-(2-Chlorbenzyliden)-acetessigsäureethylester mit 6-Amino-2.4-morpholino-pyrimidin in Ethanol der 2.4-Dimorpholino-7-methyl-5-(2-chlorphenyl)-5.8-dihydro-pyrido[2.3-d]pyrimidin-6-carbonsäureethylester vom Fp. : 241 °C (Ethanol) erhalten. Ausbeute : 84 %.

Beispiel 56

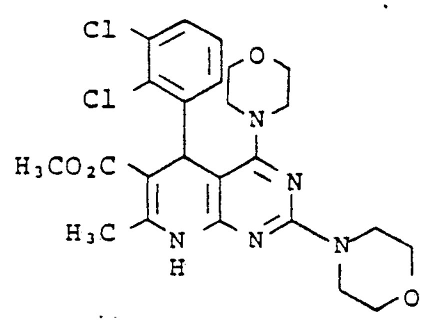

Analog Beispiel 1 wurde durch Umsetzung von 2-(2.3-Dichlorbenzyliden)-acetessigsäuremethylester mit 6-Amino-2.4-dimorpholino-pyrimidin in Ethanol der 2.4-Dimorpholino-7-methyl-5-(2.3-dichlorphenyl)-5.8-dihydro-pyrido[2.3-d]pyrimidin-6-carbonsäuremethylester vom Fp. : 188 °C (Ethanol) erhalten. Ausbeute : 85 %.

Beispiel 57

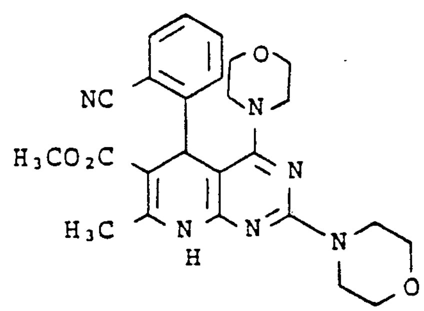

Analog Beispiel 1 wurde durch Umsetzung von 2-(2-Cyanobenzyliden)-acetessigsäuremethylester mit 6-Amino-2.4-dimorpholino-pyrimidin in Ethanol der 2.4-Dimorpholino-7-methyl-5-(2-cyanophenyl)-5.8-dihydro-pyrido[2.3-d]pyrimidin-6-carbonsäuremethylester vom Fp. : 221 °C (Ethanol) erhalten. Ausbeute : 69 %.

Beispiel 58

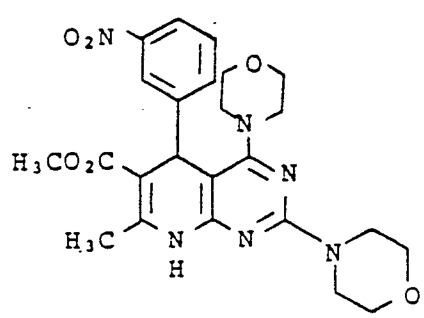

Analog Beispiel 1 wurde durch Umsetzung von 2-(3-Nitrobenzyliden)-acetessigsäuremethylester mit 6-Amino-2.4-dimorpholino-pyrimidin in Ethanol der 2.4-Dimorpholino-7-methyl-5-(3-nitrophenyl)-5.8-dihydro-pyrido[2.3-d]pyrimidin-6-carbonsäuremethylester vom Fp. : 165 °C (Ethanol) erhalten. Ausbeute : 86 %.

Beispiel 59

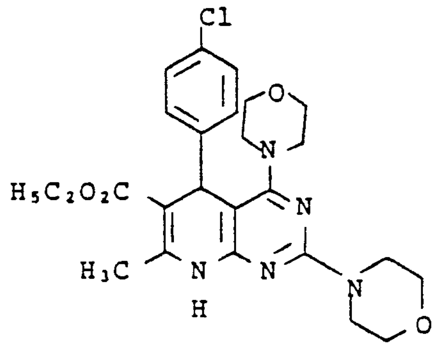

Analog Beispiel 1 wurde durch Umsetzung von 2-(4-Chlorbenzyliden)-acetessigsäureethylester mit 6-Amino-2.4-dimorpholino-pyrimidin in Ethanol der 2.4-Dimorpholino-7-methyl-5-(4-chlorphenyl)-5.8-dihydro-pyrido[2.3-d]pyrimidin-6-carbonsäureethylester vom Fp. : 128 °C (Isopropanol) erhalten. Ausbeute : 65 %.

Beispiel 60

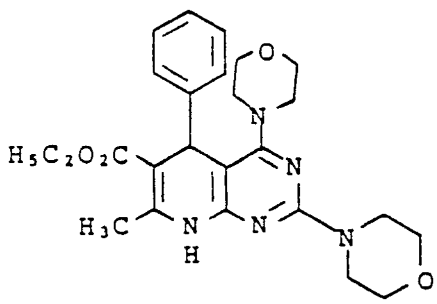

Analog Beispiel 1 wurde durch Umsetzung von 2-Benzylidenacetessigsäureethylester mit 6-Amino-2.4-dimorpholino-pyrimidin in Ethanol der 2.4-Dimorpholino-7-methyl-5-phenyl-5.8-dihydro-pyrido[2.3-d]pyrimidin-6-carbonsäureethylester vom Fp. : 178 °C (Ethanol) erhalten. Ausbeute : 82 %.

Beispiel 61

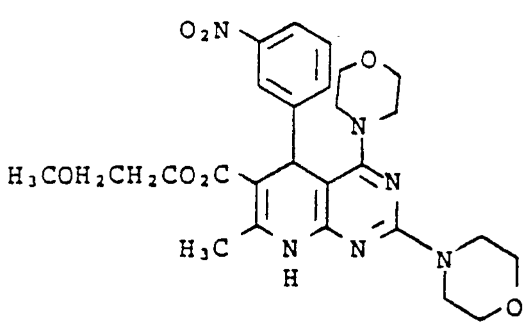

Analog Beispiel 1 wurde durch Umsetzung von 2-(3-Nitrobenzyliden)-acetessigsäure-(2-methoxyethyl)-ester mit 6-Amino-2.4-dimorpholino-pyrimidin in Ethanol der 2.4-Dimorpholino-7-methyl-5-(3-nitrophenyl)-5.8-dihydro-pyrido[2.3-d]pyrimidin-6-carbonsäure-(2-methoxyethyl)-ester vom Fp. : 172 °C (Ethanol) erhalten. Ausbeute : 93 %.

Beispiel 62

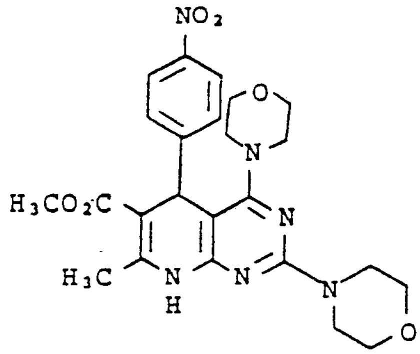

Analog Beispiel 1 wurde durch Umsetzung von 2-(4-Nitrobenzyliden)-acetessigsäuremethylester mit 6-Amino-2.4-dimorpholino-pyrimidin in Ethanol der 2.4-Dimorpholino-7-methyl-5-(4-nitrophenyl)-5.8-dihydro-pyrido[2.3-d]pyrimidin-6-carbonsäuremethylester vom Fp. : 236 °C (Ethanol) erhalten. Ausbeute : 81 %.

Beispiel 63

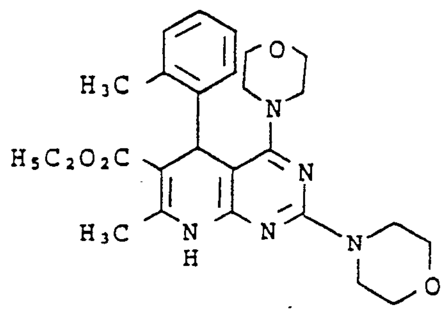

Analog Beispiel 1 wurde durch Umsetzung von 2-(2-Methylbenzyliden)-acetessigsäureethylester mit 6-Amino-2.4-dimorpholino-pyrimidin in Ethanol der 2.4-Dimorpholino-7-methyl-5-(2-methylphenyl)-5.8-dihydro-pyrido[2.3-d]pyrimidin-6-carbonsäureethylester vom Fp. : 234 °C (Ethanol) erhalten. Ausbeute : 79 %.

Beispiel 64

2.4-Dimorpholino-7-methyl-5-(3-cyanophenyl)-5.8-dihydro-pyrido[2.3-d]pyrimidin-6-carbonsäureethylester

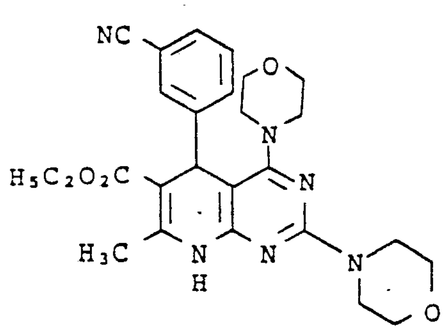

0.8 g (6 mMol) 3-Cyanobenzaldehyd, 0.8 g (6 mMol) Acetessigsäureethylester und 1.6 g (6 mMol) 6-Amino-2.4-dimorpholino-pyrimidin wurden zusammen in 20 ml Ethanol 15 Stunden unter Rückfluß erhitzt. Anschließend wurde die Reaktionsmischung abgekühlt, das ausgefallene Produkt abgesaugt und aus wenig Ethanol umkristallisiert, Fp. : 210 °C. Ausbeute : 1.8 g (61 %).

Beispiel 65

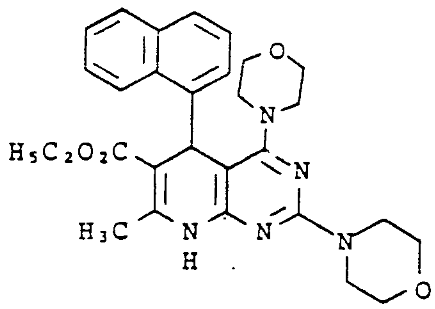

Analog Beispiel 64 wurde durch Umsetzung von α-Naphthylaldehyd, Acetessigsäureethylester und 6-Amino-2.4-dimorpholino-pyrimidin in Ethanol der 2.4-Dimorpholino-7-methyl-5-(naphthyl-1)-5.8-dihydro-pyrido[2.3-d]pyrimidin-6-carbonsäureethylester vom Fp.: 198 °C (Essigester) erhalten. Ausbeute: 58 %.

Beispiel 66

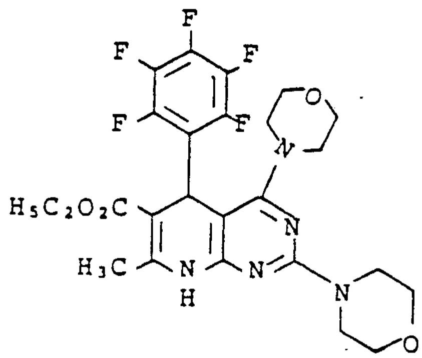

Analog Beispiel 64 wurde durch Umsetzung von Pentafluorbenzaldehyd, Acetessigsäureethylester und 6-Amino-2.4-dimorpholino-pyrimidin in Ethanol der 2.4-Dimorpholino-7-methyl-5-(pentafluorphenyl)-5.8-dihydro-pyrido[2.3-d]pyrimidin-6-carbonsäureethylester vom Fp.: 195 °C (Ethanol) erhalten. Ausbeute: 65 %.

Beispiel 67

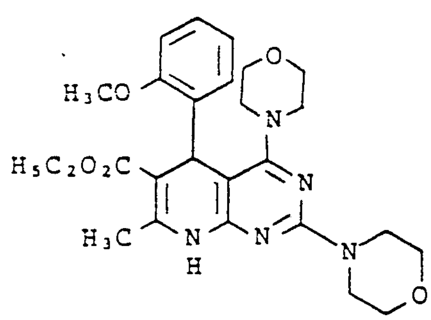

Analog Beispiel 64 wurde durch Umsetzung von 2-Methoxybenzaldehyd, Acetessigsäureethylester und 6-Amino-2.4-dimorpholino-pyrimidin in Ethanol der 2.4-Dimorpholino-7-methyl-5-(2-methoxyphenyl)-5.8-dihydro-pyrido[2.3-d]pyrimidin-6-carbonsäureethylester vom Fp.: 188 °C (Ethanol) erhalten. Ausbeute: 81 %.

(Siehe Formel Seite 29 f.)

Beispiel 68

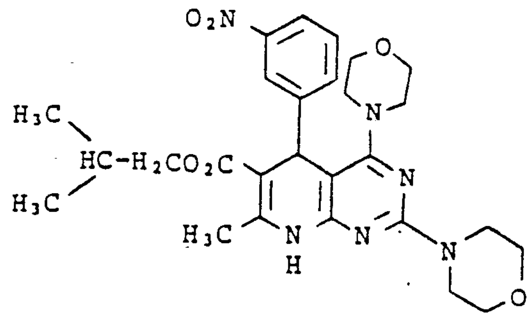

Analog Beispiel 64 wurde durch Umsetzung von 3-Nitrobenzaldehyd, Acetessigsäureisobutylester und 6-Amino-2.4-dimorpholino-pyrimidin in Ethanol der 2.4-Dimorpholino-7-methyl-5-(3-nitrophenyl)-5.8-dihydro-pyrido[2.3-d]pyrimidin-6-carbonsäureisobutylester vom Fp. : 214 °C (Ethanol) erhalten. Ausbeute : 66 %.

Beispiel 69

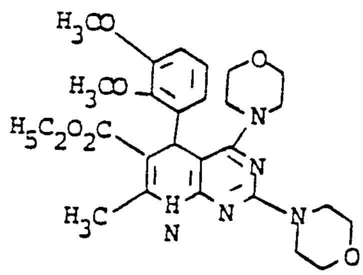

Analog Beispiel 64 wurde durch Umsetzung von 2,3-Dimethoxybenzaldehyd, Acetessigsäureethylester und 6-Amino-2,4-dimorpholino-pyrimidin in Ethanol der 2,4-Dimorpholino-7-methyl-5-(2.3-dimethoxyphenyl)-5,8-dihydro-pyrido[2,3-d]pyrimidin-6-carbonsäureethylester vom Fp : 194 °C (Ethanol) erhalten. Ausbeute : 57 %.

Beispiel 70

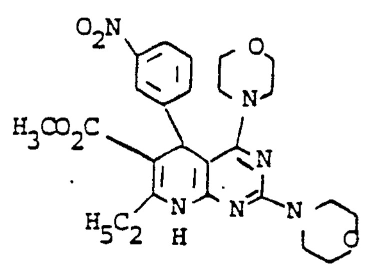

Analog Beispiel 64 wurde durch Umsetzung von 3-Nitrobenzaldehyd, Propionylessigsäuremethylester und 6-Amino-2,4-dimorpholino-pyrimidin in Ethanol der 2,4-Dimorpholino-7-ethyl-5-(3-nitrophenyl)-5,8-dihydro-pyrido[2,3-d]pyrimidin-6-carbonsäuremethylester vom Fp. 225 °C (Chloroform/Essigester) erhalten. Ausbeute : 51 %.

Beispiel 71

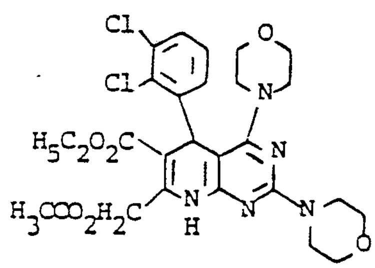

Analog Beispiel 64 wurde durch Umsetzung von 2,3-Dichlorbenzaldehyd, 4-Acetoxyacetessigsäureethylester und 6-Amino-2,4-dimorpholino-pyrimidin in Ethanol der 2,4-Dimorpholino-7-acetoxymethyl-5-(2,3-dichlorphenyl)-5,8-dihydro-pyrido[2,3-d]pyrimidin-6-carbonsäureethylester vom Fp : 161 °C (Chloroform/Essigester) erhalten. Ausbeute : 47 %.

Beispiel 72

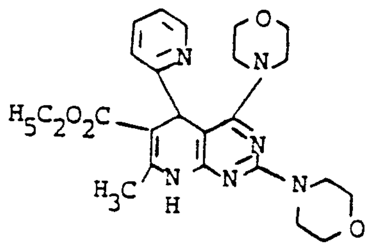

Analog Beispiel 64 wurde durch Umsetzung vom Pyridin-2-aldehyd, Acetessigsäureethylester und 6-Amino-2,4-dimorpholino-pyrimidin in Ethanol der 2,4-Dimorpholino-7-methyl-5-(pyridyl-2)-5,8-dihydro-pyrido[2,3-d]pyrimidin-6-carbonsäure-ethylester vom Fp : 226 °C (Ethanol/Dimethylformamid) erhalten. Ausbeute : 53 %.

Beispiel 73

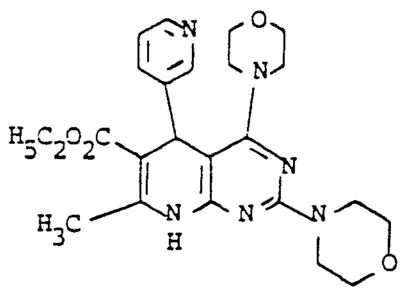

Analog Beispiel 64 wurde durch Umsetzung von Pyridin-3-aldehyd, Acetessigsäureethylester und 6-Amino-2,4-dimorpholino-pyrimidin in Ethanol der 2,4-Dimorpholino-7-methyl-5-(pyridyl-3)-5,8-dihydro-pyrido[2,3-d]pyrimidin-6-carbonsäureethylester vom Fp : 229 °C (Ethanol) erhalten. Ausbeute : 56 %.

Beispiel 74

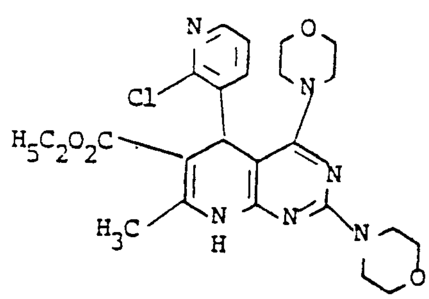

Analog Beispiel 64 wurde durch Umsetzung von 2-Chlorpyridin-3-aldehyd, Acetessigsäureethylester und 6-Amino-2,4-dimorpholino-pyrimidin in Ethanol der 2,4-Dimorpholino-7-methyl-5-(2-chlor-pyridyl-3)-5,8-dihydro-pyrido[2,3-d]pyrimidin-6-carbonsäureethylester vom Fp : 185 °C, (Essigester) erhalten. Ausbeute : 62 %.

Beispiel 75

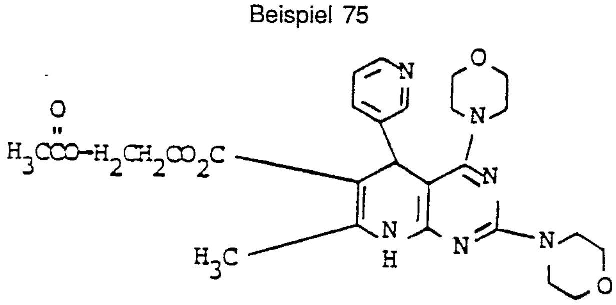

Analog Beispiel 64 wurde durch Umsetzung von Pyridin-3-aldehyd, Acetessigsäure-(2-acetoxyethyl)-ester und 6-Amino-2,4-dimorpholino-pyrimidin in Isopropanol der 2,4-Dimorpholino-7-methyl-5-(pyridyl-3)-5,8-dihydro-pyrido[2,3-d]pyrimidin-6-carbonsäure-(2-acetoxyethyl)-ester vom Fp : 170 °C (Ethanol) erhalten. Ausbeute : 59 %.

Beispiel 76

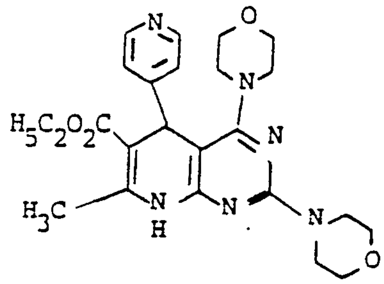

Analog Beispiel 64 wurde durch Umsetzung von Pyridin-4-aldehyd, Acetessigsäureethylester und 6-Amino-2,4-dimorpholino-pyrimidin in Ethanol der 2,4-Dimorpholino-7-methyl-5-(pyridyl-4)-5,8-dihydro-pyrido[2,3-d]pyrimidin-6-carbonsäureethylester vom Fp : 209 °C (Toluol) erhalten. Ausbeute : 53 %.

Beispiel 77

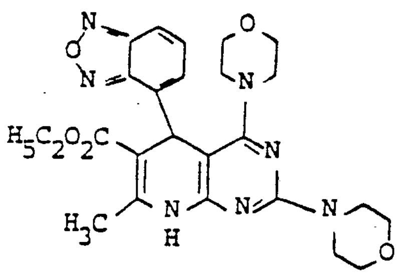

Analog Beispiel 64 wurde durch Umsetzung von 2,1,3-Benzoxadiazol-4-aldehyd, Acetessigsäureethylester und 6-Amino-2,4-dimorpholino-pyrimidin in Ethanol der 2,4-Dimorpholino-7-methyl-5-(2,1,3-benzoxadiazolyl-4)-5,8-dihydro-pyrido[2,3-d]pyrimidin-6-carbonsäureethylester vom Fp : 234 °C (Essigester) erhalten. Ausbeute : 49 %.

Beispiel 78

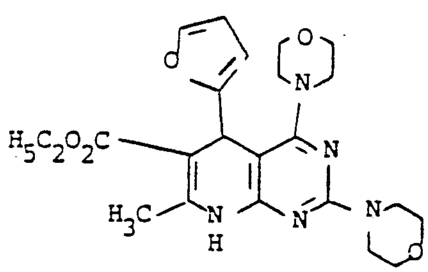

Analog Beispiel 64 wurde durch Umsetzung von Furan-2-aldehyd, Acetessigsäureethylester und 6-Amino-2,4-dimorpholino-pyrimidin in Ethanol der 2,4-Dimorpholino-7-methyl-5-(furyl-2)-5,8-dihydro-pyrido[2,3-d]pyrimidin-6-carbonsäureethylester vom Fp : 172 °C (Toluol) erhalten. Ausbeute : 56 %.

Beispiel 79

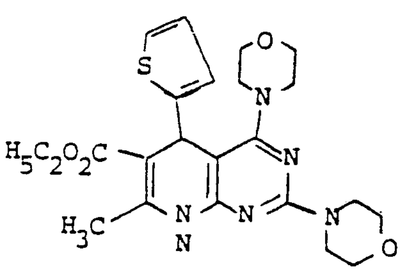

Analog Beispiel 64 wurde durch Umsetzung von Thiophen-2-aldehyd, Acetessigsäureethylester und 6-Amino-2,4-dimorpholino-pyrimidin in Ethanol der 2,4-Dimorpholino-7-methyl-5-(thienyl-2)-5,8-dihydro-pyrido[2,3-d]pyrimidin-6-carbonsäure-ethylester vom Fp : 205 °C (Essigester) erhalten. Ausbeute : 43 %.

Beispiel 80

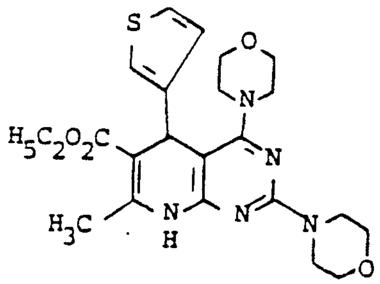

Analog Beispiel 64 wurde durch Umsetzung von Thiophen-3-aldehyd, Acetessigsäureethylester und 6-Amino-2,4-dimorpholino-pyrimidin in Ethanol der 2,4-Dimorpholino-7-methyl-5-(thienyl-3)-5,8-dihydro-pyrido[2,3-d]pyrimidin-6-carbonsäureethylester vom Fp : 208 °C (Ethanol) erhalten. Ausbeute : 69 %.

**Patentansprüche**

1. Pyridopyrimidine der allgemeinen Formel (I)

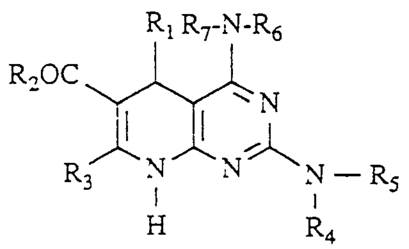

in welcher

$R_1$ für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisooxazolyl, Benzoxadiazolyl, Benzthiadiazolyl, Chinolyl, Isochinolyl, Chinazolyl oder Chinoxalyl steht, wobei die genannten Heterocyclen sowie der Phenylrest und der Naphthylrest jeweils 1 bis 5 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest, Carbamoyl, N.N-Dimethylcarbamoyl, Sulfamoyl, $SO_m$-Alkyl, worin m 0 bis 2 bedeutet und Alkyl 1 bis 4 Kohlenstoffatome enthält, oder $SO_m$-Benzyl mit m = 0 bis 2 aufgeführt seien,

$R_2$ für einen geradkettigen, verzweigten oder cyclischen Alkylrest mit bis zu 8 Kohlenstoffalomen oder für eine Phenyl- oder Benzylgruppe steht,

oder

für eine Amino-, Mono- oder Dialkylaminogruppe mit bis zu 4 Kohlenstoffatomen je Alkylgruppe steht, wobei eine oder beide Alkylgruppen durch einen Phenylrest substituiert sein können,

oder

für einen Anilinorest steht, der gegebenenfalls durch Fluor oder Chlor, oder durch Alkyl oder Alkoxy mit 1 bis 4 Kohlenstoffatomen substituiert ist,

oder

für den Rest $OR_8$ steht, wobei $R_8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Benzoyloxy, Nitro, Nitrooxy, Alkoxycarbonyl mit bis zu 4 Kohlenstoffatomen in der Alkoxygruppe, Phenyl, Phenoxy, Phenylthio, Phenylsulfonyl, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 Kohlenstoffatomen, Alkoxyalkyl mit bis zu 6

Kohlenstoffatomen, Phenyl, Benzyl oder Phenethyl trägt oder wobei der Stickstoff dieser Aminogruppe gegebenenfalls mit den Substituenten einen 5- bis 7-gliedrigen Ring bildet, die als weiteres Heteroatom ein Sauerstoff- oder Schwefelatom oder eine N-Phenylgruppe oder eine N-Alkylgruppe mit 1 bis 4 Kohlenstoffatomen im Alkylrest enthalten kann,

$R_3$ für Wasserstoff steht

oder

für einen geradkettigen, verzweigten oder cyclischen Kohlenwasserstoffrest mit bis zu 6 Kohlenstoffatomen steht, der gegebenenfalls durch 1 oder 2 Alkoxygruppen mit 1 bis 4 Kohlenstoffatomen oder eine Acetoxy- oder Benzoyloxygruppe substituiert ist,

oder

für einen Phenylrest oder einen Benzylrest steht,

und

$R_4$, $R_5$, $R_6$ und $R_7$ gleich oder verschieden sind und jeweils für Wasserstoff stehen

oder

für einen gegebenenfalls durch Hydroxy, Methoxy, Acetoxy oder Benzoyloxy substituierten geradkettigen oder verzweigten Alkylrest mit bis zu 8 Kohlenstoffatomen stehen, wobei die Reste $R_4$, $R_5$ sowie $R_6$ und $R_7$ mit dem jeweiligen Stickstoffatom einen 5 bis 7 gliedrigen Ring bilden können, der gegebenenfalls als weiteres Heteroatom Sauerstoff oder Schwefel oder eine N-Alkylgruppe mit bis zu 4 Kohlenstoffatomen oder eine N-Phenylgruppe enthält,

oder

für einen Phenyl- oder Benzylrest stehen.

2. Verbindungen der allgemeinen Formel (I) gemäß Anspruch 1, in welcher

$R_1$ für einen Phenyl- oder Naphthylrest oder für Thienyl, Furyl, Pyridyl, Pyridazinyl, Pyrimidyl, Benzoxadiazolyl, Benzthiadiazolyl, Chinolyl oder Isochinolyl steht, wobei die genannten Heterocyclen sowie der Phenyl- und der Naphthylrest 1 bis 5 gleiche oder verschiedene Substituenten tragen können, aus der Gruppe geradkettiges oder verzweigtes Alkyl mit bis zu 8 Kohlenstoffatomen, Alkoxyalkyl mit 1 bis 4 Kohlenstoffatomen, Dioxymethylen, Fluor, Chlor, Brom, Trifluormethyl, Trifluormethoxy, Difluormethoxy, Nitro, Cyano, Azido, Thioalkyl, Sulfonylalkyl mit jeweils 1 bis 4 C-Atomen im Alkylrest, Thiobenzyl und Sulfonylbenzyl,

$R_2$ für Alkyl mit bis zu 8 C-Atomen, Phenyl oder Benzyl steht,

oder

für den Rest $OR_8$ steht, wobei $R_8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Phenyl, Phenoxy, Phenylthio, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 C-Atomen, Phenyl oder Benzyl trägt,

$R_3$ für einen geradkettigen oder verzweigten Kohlenwasserstoffrest mit bis zu 6 C-Atomen steht, der gegebenenfalls substituiert ist durch 1 oder 2 Alkoxygruppen mit 1 bis 4 C-Atomen oder eine Acetoxygruppe,

oder

für einen Phenylrest oder Benzylrest steht, und

$R_4$, $R_5$, $R_6$ und $R_7$ jeweils gleich oder verschieden sind und für Wasserstoff oder einen geradkettigen oder verzweigten Alkylrest mit bis zu 8 Kohlenstoffatomen stehen, der gegebenenfalls substituiert ist durch Hydroxy, Methoxy oder Acetoxy und wobei die Reste $R_4$ und $R_5$ sowie $R_6$ und $R_7$ mit dem jeweiligen Stickstoffatomen einen 5 bis 7 gliedrigen Ring bilden können, der gegebenenfalls als weiteres Heteroatom Sauerstoff, Schwefel oder eine N-Alkylgruppe mit bis zu 4 C-Atomen oder eine N-Phenylgruppe enthält.

3. Verfahren zur Herstellung von Verbindungen der allgemeinen Formel (I)

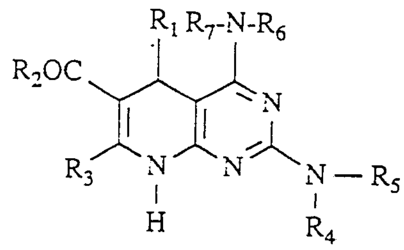

in welcher

$R_1$ für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisooxazolyl, Benzoxadiazolyl, Benzthiadiazolyl, Chinolyl, Isochinolyl, Chinazolyl oder Chinoxalyl steht, wobei die genannten Heterocyclen sowie der Phenylrest und der Naphthylrest jeweils 1 bis 5 gleiche oder verschiedene Substituenten tragen können, wobei als Substituenten Phenyl, geradkettiges

oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest, Carbamoyl, N.N-Dimethylcarbamoyl, Sulfamoyl, $SO_m$-Alkyl, worin m 0 bis 2 bedeutet und Alkyl 1 bis 4 Kohlenstoffatome enthält, oder $SO_m$-Benzyl mit m = 0 bis 2 aufgeführt seien,

$R_2$ für einen geradkettigen, verzweigten oder cyclischen Alkylrest mit bis zu 8 Kohlenstoffatomen oder für eine Phenyl- oder Benzylgruppe steht,

oder

für eine Amino-, Mono- oder Dialkylaminogruppe mit bis zu 4 Kohlenstoffatomen je Alkylgruppe steht, wobei eine oder beide Alkylgruppen durch einen Phenylrest substituiert sein können,

oder

für einen Anilinorest steht, der gegebenenfalls durch Fluor oder Chlor, oder durch Alkyl oder Alkoxy mit 1 bis 4 Kohlenstoffatomen substituiert ist,

oder

für den Rest $OR_8$ steht, wobei $R_8$ für einen geradkettigen, verzweigten oder cyclischen, gesättigten oder ungesättigten Kohlenwasserstoffrest mit bis zu 12 Kohlenstoffatomen steht, der gegebenenfalls durch 1 Sauerstoff- oder Schwefelatom in der Kette unterbrochen ist und/oder der gegebenenfalls substituiert ist durch Halogen, Cyano, Hydroxy, Acetoxy, Benzoyloxy, Nitro, Nitrooxy, Alkoxycarbonyl mit bis zu 4 Kohlenstoffatomen in der Alkoxygruppe, Phenyl, Phenoxy, Phenylthio, Phenylsulfonyl, α-, β-, γ-Pyridyl oder eine Aminogruppe, wobei diese Aminogruppe gegebenenfalls ein oder zwei gleiche oder verschiedene Substituenten aus der Gruppe Alkyl mit 1 bis 4 Kohlenstoffatomen, Alkoxyalkyl mit bis zu 6 Kohlenstoffatomen, Phenyl, Benzyl oder Phenethyl trägt oder wobei der Stickstoff dieser Aminogruppe gegebenenfalls mit den Substituenten einen 5- bis 7-gliedrigen Ring bildet, die als weiteres Heteroatom ein Sauerstoff- oder Schwefelatom oder eine N-Phenylgruppe oder eine N-Alkylgruppe mit 1 bis 4 Kohlenstoffatomen im Alkylrest enthalten kann,

$R_3$ für Wasserstoff steht

oder

für einen geradkettigen, verzweigten oder cyclischen Kohlenwasserstoffrest mit bis zu 6 Kohlenstoffatomen steht, der gegebenenfalls durch 1 oder 2 Alkoxygruppen mit 1 bis 4 Kohlenstoffatomen oder eine Acetoxy- oder Benzoyloxygruppe substituiert ist,

oder

für einen Phenylrest oder einen Benzylrest steht,

und

$R_4$, $R_5$, $R_6$ und $R_7$ gleich oder verschieden sind und jeweils für Wasserstoff stehen

oder

für einen gegebenenfalls durch Hydroxy, Methoxy, Acetoxy oder Benzoyloxy substituierten geradkettigen oder verzweigten Alkylrest mit bis zu 8 Kohlenstoffatomen stehen, wobei die Reste $R_4$, $R_5$ sowie $R_6$ und $R_7$ mit dem jeweiligen Stickstoffatom einen 5 bis 7 gliedrigen Ring bilden können, der gegebenenfalls als weiteres Heteroatom Sauerstoff oder Schwefel oder eine N-Alkylgruppe mit bis zu 4 Kohlenstoffatomen oder eine N-Phenylgruppe enthält,

oder

für einen Phenyl- oder Benzylrest stehen,

dadurch gekennzeichnet, daß man

A) Yliden-β-dicarbonylverbindungen der allgemeinen Formel (II)

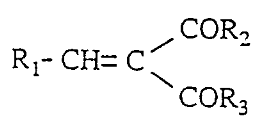

in welcher

$R_1$, $R_2$ und $R_3$ die oben angegebene Bedeutung haben mit 6-Aminopyrimidinen der allgemeinen Formel III,

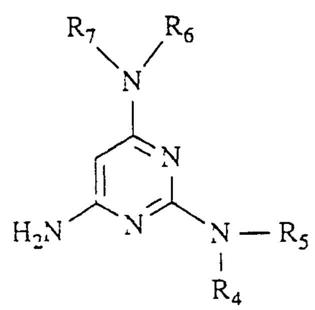

in welcher

$R_4$, $R_5$, $R_6$ und $R_7$ die oben angegebene Bedeutung haben, gegebenenfalls in Gegenwart organischer Lösungsmittel umsetzt,

oder

B) Aldehyde der allgemeinen Formel IV

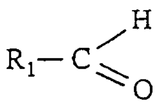

in welcher

$R_1$ die oben angegebene Bedeutung hat, mit β-Dicarbonylverbindungen der allgemeinen Formel V

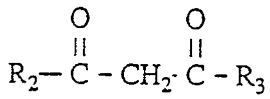

in welcher

$R_2$ und $R_3$ die oben angegebene Bedeutung haben, und 6-Aminopyrimidinen der allgemeinen Formel III

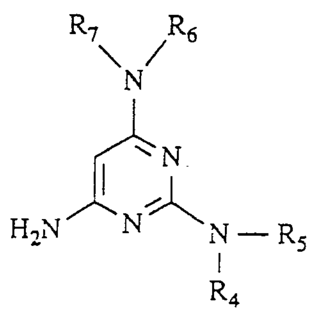

in welcher

$R_4$, $R_5$, $R_6$ und $R_7$ die oben angegebene Bedeutung haben, gegebenenfalls in Gegenwart von inerten organischen Lösungsmitteln bei Temperaturen zwischen 20 und 150 °C umsetzt.

4. Verbindungen der allgemeinen Formel (I) gemäß Anspruch 1 zur Verwendung bei der Bekämpfung von Kreislauferkrankungen.

5. Arzneimittel enthaltend mindestens eine Verbindung aus der allgemeinen Formel (I) gemäß Anspruch 1.

6. Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von Hilfs- und Trägerstoffen in eine geeignete Applikationsform überführt.

7. Verwendung von Verbindungen der allgemeinen Formel (I) gemäß Anspruch 1 bei der Herstellung von Kreislaufmitteln.

**Claims**

1. Pyridopyrimidines of the general formula (I)

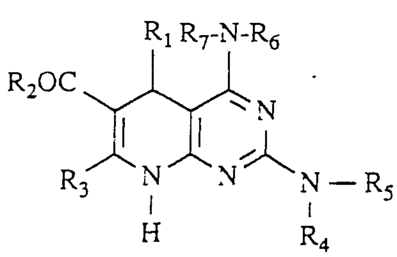

in which

$R_1$ represents a phenyl or naphthyl radical or thienyl, furyl, pyrryl, pyrazolyl, imidazolyl, oxazolyl, isoxazolyl, thiazolyl, pyridyl, pyridazinyl, pyrimidyl, pyrazinyl, indolyl, benzimidazolyl, benzoxazolyl, benzisoxazolyl, benzoxadiazolyl, benzthiadiazolyl, quinolyl, isoquinolyl, quinazolyl or quinoxalyl, it being possible for the heterocyclic radicals mentioned and the phenyl radical and the naphthyl radical each to carry 1 to 5 identical or different substituents, for which phenyl, straight-chain or branched alkyl having

up to 8 carbon atoms, cycloalkyl having 3 to 7 carbon atoms, alkenyl or alkinyl having 2 to 6 carbon atoms, alkenoxy and alkinoxy having 2 to 6 carbon atoms, tri-, tetra- and penta-methylene, dioxymethylene, dioxyethylidene, halogen, such as fluorine, chlorine, bromine or iodine, trifluoromethyl, trifluoroethyl, trifluoromethoxy, difluoromethoxy, tetrafluoroethoxy, dialkylamino having 1 to 4 C atoms, nitro, cyano, azido, alkoxycarbonyl having 1 to 4 C atoms in the alkoxy radical carbamoyl N,N-dimethylcarbamoyl, sulphamoyl, $SO_m$-alkyl, in which m denotes 0 to 2 and alkyl contains 1 to 4 carbon atoms, or $SO_m$-benzyl with m = 0 to 2 may be mentioned as substituents,

$R_2$ represents a straight-chain, branched or cyclic alkyl radical having up to 8 carbon atoms, or a phenyl or benzyl group,

or

represents an amino, monoamino or dialkylamino group having up to 4 carbon atoms per alkyl group, it being possible for one or both alkyl groups to be substituted by a phenyl radical,

or

represents an anilino radical optionally substituted by fluorine or chlorine or by alkyl or alkoxy having 1 to 4 carbon atoms,

or

represents the radical $OR_8$, in which $R_8$ represents a straight-chain, branched or cyclic, saturated or unsaturated hydrocarbon radical which has up to 12 carbon atoms and is optionally interrupted by 1 oxygen or sulphur atom in the chain and/or is optionally substituted by halogen, cyano, hydroxyl, acetoxy, benzoyloxy, nitro, nitrooxy, alkoxycarbonyl having up to 4 carbon atoms in the alkoxy group, phenyl, phenoxy, phenylthio, phenylsulphonyl, α-, β- or γ-pyridyl or an amino group, this amino group optionally carrying one or two identical or different substituents from the group comprising alkyl having 1 to 4 carbon atoms, alkoxyalkyl having up to 6 carbon atoms, phenyl, benzyl or phenethyl, or the nitrogen of this amino group, together with the substituents, optionally forming a 5-membered to 7-membered ring which can contain an oxygen or sulphur atom as a further heteroatom or an N-phenyl group or an N-alkyl group having 1 to 4 carbon atoms in the alkyl radical,

$R_3$ represents hydrogen

or

represents a straight-chain, branched or cyclic hydrocarbon radical which has up to 6 carbon atoms and is optionally substituted by 1 or 2 alkoxy groups having 1 to 4 carbon atoms or by an acetoxy or benzoyloxy group,

or

represents a phenyl radical or a benzyl radical, and

$R_4$, $R_5$, $R_6$ and $R_7$ are identical or different and each represent hydrogen

or

represent a straight-chain or branched alkyl radical which has up to 8 carbon atoms and is optionally substituted by hydroxyl, methoxy, acetoxy or benzoyloxy, it being possible for the radicals $R_4$ and $R_5$ and/or $R_6$ and $R_7$, together with the respective nitrogen atom, to form a 5-membered to 7-membered ring which optionally contains, as a further heteroatom, oxygen or sulphur or an N-alkyl group having up to 4 carbon atoms or an N-phenyl group,

or

represent a phenyl or benzyl radical.

2. Compounds of the general formula (I) according to Claim 1, in which

$R_1$ represents a phenyl or naphthyl radical or thienyl, furyl, pyridyl, pyridazinyl, pyrimidyl, benzoxadiazolyl, benzthiadiazolyl, quinolyl or isoquinolyl, it being possible for the said heterocyclic radicals and for the phenyl and naphthyl radical to carry 1 to 5 identical or different substituents from the group comprising straight-chain or branched alkyl having up to 8 carbon atoms, alkoxyalkyl having 1 to 4 carbon atoms, dioxymethylene, fluoro, chloro, bromo, trifluoromethyl, trifluoromethoxy, difluoromethoxy, nitro, cyano, azido, thioalkyl, sulphonylalkyl each having 1 to 4 C atoms in the alkyl radical, thiobenzyl and sulphonylbenzyl,

$R_2$ represents alkyl having up to 8 C atoms, phenyl or benzyl,

or

represents the radical $OR_8$, in which $R_8$ represents a straight-chain branched or cyclic, saturated or unsaturated hydrocarbon radical which has up to 12 carbon atoms and is optionally interrupted by 1 oxygen or sulphur atom in the chain and/or is optionally substituted by halogen, cyano, hydroxyl, acetoxy, phenyl, phenoxy, phenylthio, α-, β-, or γ-pyridyl or an amino group, this amino group optionally carrying one or two identical or different substituents from the group comprising alkyl having 1 to 4 C atoms, phenyl and benzyl,

$R_3$ represents a straight-chain or branched hydrocarbon radical which has up to 6 C atoms and is optionally substituted by 1 or 2 alkoxy groups having 1 to 4 C atoms or by an acetoxy group,

or

represents a phenyl radical or benzyl radical and

$R_4$, $R_5$, $R_6$ and $R_7$ each are identical or different and represent hydrogen or a straight-chain or branched alkyl radical which has up to 8 carbon atoms and is optionally substituted by hydroxyl, methoxy or acetoxy, it being possible for the radicals $R_4$ and $R_5$ and/or $R_6$ and $R_7$, together with the respective

nitrogen atom, to form a 5-membered to 7-membered ring which optionally contains, as a further heteroatom, oxygen, sulphur or an N-alkyl group having up to 4 C atoms or an N-phenyl group.

3. Process for the preparation of compounds of the general formula (I)

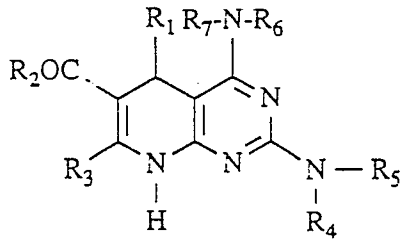

in which

$R_1$ represents a phenyl or naphthyl radical or thienyl, furyl, pyrryl, pyrazolyl, imidazolyl, oxazolyl, isoxazolyl, thiazolyl, pyridyl, pyridazinyl, pyrimidyl, pyrazinyl, indolyl, benzimidazolyl, benzoxazolyl, benzisoxazolyl, benzoxadiazolyl, benzthiadiazolyl, quinolyl, isoquinolyl, quinazolyl or quinoxalyl, it being possible for the heterocyclic radicals mentioned and the phenyl radical and the naphthyl radical each to carry 1 to 5 identical or different substituents, for which phenyl, straight-chain or branched alkyl having up to 8 carbon atoms, cycloalkyl having 3 to 7 carbon atoms, alkenyl or alkinyl having 2 to 6 carbon atoms, alkenoxy and alkinoxy having 2 to 6 carbon atoms, tri-, tetra- and pentamethylene, dioxymethylene, dioxyethylidene, halogen, such as fluorine, chlorine, bromine or iodine, trifluoromethyl, trifluoroethyl, trifluoromethoxy, difluoromethoxy, tetrafluoroethoxy, dialkylamino having 1 to 4 C atoms, nitro, cyano, azido, alkoxycarbonyl having 1 to 4 C atoms in the alkoxy radical, carbamoyl, N,N-dimethylcarbamoyl, sulphamoyl, $SO_m$-alkyl, in which m denotes 0 to 2 and alkyl contains 1 to 4 carbon atoms, or $SO_m$-benzyl with m = 0 to 2 may be mentioned as substituents,

$R_2$ represents a straight-chain, branched or cyclic alkyl radical having up to 8 carbon atoms, or a phenyl or benzyl group,

or

represents an amino, monoamino or dialkylamino group having up to 4 carbon atoms per alkyl group, it being possible for one or both alkyl groups to be substituted by a phenyl radical,

or

represents an anilino radical optionally substituted by fluorine or chlorine or by alkyl or alkoxy having 1 to 4 carbon atoms,

or

represents the radical $OR_8$, in which $R_8$ represents a straight-chain, branched or cyclic, saturated or unsaturated hydrocarbon radical which has up to 12 carbon atoms and is optionally interrupted by 1 oxygen or sulphur atom in the chain and/or is optionally substituted by halogen, cyano, hydroxyl, acetoxy, benzoyloxy, nitro, nitrooxy, alkoxycarbonyl having up to 4 carbon atoms in the alkoxy group, phenyl, phenoxy, phenylthio, phenylsulphonyl, α-, β- or γ-pyridyl or an amino group, this amino group optionally carrying one or two identical or different substituents from the group comprising alkyl having 1 to 4 carbon atoms, alkoxyalkyl having up to 6 carbon atoms, phenyl, benzyl or phenethyl, or the nitrogen of this amino group, together with the substituents, optionally forming a 5-membered to 7-membered ring which can contain an oxygen or sulphur atom as a further heteroatom or an N-phenyl group or an N-alkyl group having 1 to 4 carbon atoms in the alkyl radical,

$R_3$ represents hydrogen

or

represents a straight-chain, branched or cyclic hydrocarbon radical which has up to 6 carbon atoms and is optionally substituted by 1 or 2 alkoxy groups having 1 to 4 carbon atoms or by an acetoxy or benzoyloxy group,

or

represents a phenyl radical or a benzyl radical,

and

$R_4$, $R_5$, $R_6$ and $R_7$ are identical or different and each represent hydrogen

or

represent a straight-chain or branched alkyl radical which has up to 8 carbon atoms and is optionally substituted by hydroxyl, methoxy, acetoxy or benzoyloxy, it being possible for the radicals $R_4$ and $R_5$ and/or $R_6$ and $R_7$, together with the respective nitrogen atom, to form a 5-membered to 7-membered ring which optionally contains, as a further heteroatom, oxygen or sulphur or an N-alkyl group having up to 4 carbon atoms or an N-phenyl group,

or

represent a phenyl or benzyl radical,

characterized in that

A) ylidene-β-dicarbonyl compounds of the general formula (II)

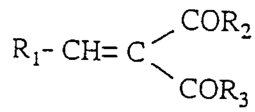

in which

$R_1$, $R_2$ and $R_3$ have the meaning given above, are reacted with 6-aminopyrimidines of the general formula III

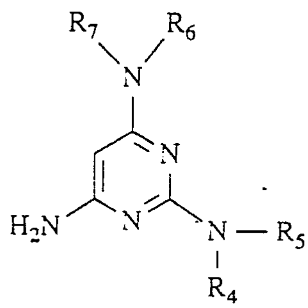

in which

$R_4$, $R_5$, $R_6$ and $R_7$ have the meaning given above, optionally in the presence of organic solvents,

or

B) aldehydes of the general formula IV

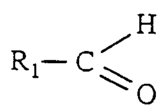

in which

$R_1$ has the meaning given above, are reacted with β-dicarbonyl compounds of the general formula V

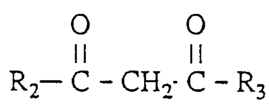

in which

$R_2$ and $R_3$ have the meaning given above, and 6-aminopyrimidines of the general formula III

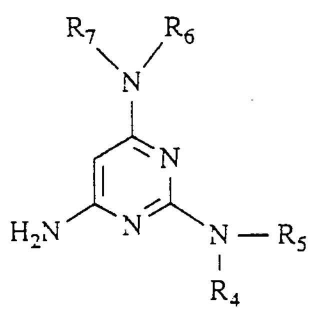

in which

$R_4$, $R_5$, $R_6$ and $R_7$ have the meaning given above, optionally in the presence of inert organic solvents at temperatures between 20 and 150 °C.

4. Compounds of the general formula (I) according to Claim 1 for use in the control of circulatory diseases.

5. Medicament containing at least one compound of the general formula (I) according to claim 1.

6. Process for the preparation of medicaments, characterized in that at least one compound of the general formula (I) according to Claim 1 is converted into a suitable administration form, optionally using auxiliaries and excipients.

7. Use of compounds of the general formula (I) according to Claim 1 in the preparation of circulatory medicaments.

## Revendications

1. Pyridopyrimidines de formule générale (I) :

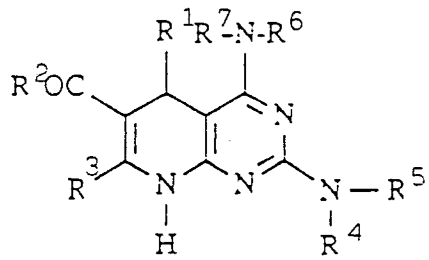

dans laquelle

$R^1$ représente un groupe phényle ou un groupe naphtyle, ou encore un groupe thiényle, un groupe furyle, un groupe pyrryle, un groupe pyrazolyle, un groupe imidazolyle, un groupe oxazolyle, un groupe isoxazolyle, un groupe thiazolyle, un groupe pyridyle, un groupe pyridazinyle, un groupe pyrimidyle, un groupe pyrazinyle, un groupe indolyle, un groupe benzimidazolyle, un groupe benzoxazolyle, un groupe benziso-oxazolyle, un groupe benzoxadiazolyle, un groupe benzthiadiazolyle, un groupe quinoléyle, un groupe isoquinoléyle, un groupe quinazolyle ou un groupe quinoxalyle ; les hétérocycles mentionnés, de même que le groupe phényle et le groupe naphtyle pouvant comporter chacun 1 à 5 substituants identiques ou différents avec comme substituants un groupe phényle, un groupe alkyle à chaîne droite ou ramifiée contenant jusqu'à 8 atomes de carbone, un groupe cycloalkyle contenant 3 à 7 atomes de carbone, un groupe alcényle ou un groupe alcynyle contenant 2 à 6 atomes de carbone, un groupe alcénoxy et un groupe alcynoxy contenant 2 à 6 atomes de carbone, un groupe tri-, tétra- et pentaméthylène, un groupe dioxyméthylène, un groupe dioxyéthylidène, un atome d'halogène tel qu'un atome de fluor, un atome de chlore, un atome de brome ou un atome d'iode, un groupe trifluorométhyle, un groupe trifluoréthyle, un groupe trifluorométhoxy, un groupe difluorométhoxy, un groupe tétrafluoréthoxy, un groupe dialkylamino contenant 1 à 4 atomes de carbone, un groupe nitro, un groupe cyano, un groupe azido, un groupe alcoxycarbonyle contenant 1 à 4 atomes de carbone dans le radical alcoxy, un groupe carbamoyle, un groupe N,N-diméthyl-carbamoyle, un groupe sulfamoyle, un groupe $SO_m$-alkyle où m représente 0 à 2, ainsi qu'un groupe alkyle contenant 1 à 4 atomes de carbone, ou un groupe $SO_m$-benzyle où m représente 0 à 2,

$R^2$ représente un groupe alkyle à chaîne droite, à chaîne ramifiée ou cyclique contenant jusqu'à 8 atomes de carbone ou un groupe phényle ou benzyle,

ou

un groupe amino, un groupe mono- ou dialkylamino contenant jusqu'à 4 atomes de carbone par groupe alkyle, un ou les deux groupes alkyle pouvant être substitués par un groupe phényle,

ou

un groupe anilino qui est éventuellement substitué par un atome de fluor ou un atome de chlore, ou par un groupe alkyle ou un groupe alcoxy contenant 1 à 4 atomes de carbone,

ou

le radical $OR^8$, $R^8$ représentant un radical d'hydrocarbure saturé ou insaturé, à chaîne droite, ramifiée ou cyclique contenant jusqu'à 12 atomes de carbone, qui est éventuellement interrompu par un atome d'oxygène ou un atome de soufre dans sa chaîne et/ou qui est éventuellement substitué par un atome d'halogène, un groupe cyano, un groupe hydroxyle, un groupe acétoxy, un groupe benzoyloxy un groupe nitro, un groupe nitrooxy un groupe alcoxycarbonyle contenant jusqu'à 4 atomes de carbone dans le groupe alcoxy, un groupe phényle, un groupe phénoxy, un groupe phénylthio, un groupe phénylsulfonyle, un groupe α-, β-, γ-pyridyle ou encore un groupe amino, ce groupe amino comportant éventuellement un ou deux substituants identiques ou différents choisis parmi le groupe comprenant un groupe alkyle contenant 1 à 4 atomes de carbone, un groupe alcoxyalkyle contenant jusqu'à 6 atomes de carbone, un groupe phényle, un groupe benzyle ou un groupe phénéthyle ou encore, l'atome d'azote de ce groupe amino formant éventuellement, avec les substituants, un noyau pentagonal à heptagonal pouvant contenir, comme autre hétéroatome, un atome d'oxygène ou un atome de soufre, ou encore un groupe N-phényle ou un groupe N-alkyle contenant 1 à 4 atomes de carbone dans le radical alkyle,

$R^3$ représente un atome d'hydrogène,

ou

un radical d'hydrocarbure à chaîne droite, chaîne ramifiée ou cyclique contenant jusqu'à 6 atomes de carbone et éventuellement substitué par un ou deux groupes alcoxy contenant 1 à 4 atomes de carbone ou un groupe acétoxy ou benzoyloxy,

ou

un groupe phényle ou un groupe benzyle,

et

$R^4$, $R^5$, $R^6$ et $R^7$ sont identiques ou différents et représentent chacun un atome d'hydrogène,

ou

un groupe alkyle à chaîne droite ou ramifiée éventuellement substitué par un groupe hydroxyle, un groupe méthoxy, un groupe acétoxy ou un groupe benzoyloxy et contenant jusqu'à 8 atomes de carbone, les radicaux $R^4$, $R^5$, ainsi que $R^6$ et $R^7$ pouvant former, avec l'atome azote correspondant un noyau pentagonal à heptagonal contenant éventuellement, comme autre hétéro-atome, un atome d'oxygène ou un atome de soufre, ou encore un groupe N-alkyle contenant jusqu'à 4 atomes de carbone, ou un groupe N-phényle,

ou

un groupe phényle ou un groupe benzyle.

2. Composés de formule générale (I) selon la revendication 1, dans laquelle

$R^1$ représente un groupe phényle ou un groupe naphtyle, ou encore un groupe thiényle, un groupe furyle, un groupe pyridyle, un groupe pyridazinyle, un groupe pyrimidyle, un groupe benzoxadiazolyle, un groupe benzthiadiazolyle, un groupe quinoléyle ou un groupe isoquinoléyle, les hétérocycle, mentionnés, de même que le groupe phényle et le groupe naphtyle pouvant comporter 1 à 5 substituants identiques ou différents choisis parmi le groupe comprenant un groupe alkyle à chaîne droite ou ramifiée contenant jusqu'à 8 atomes de carbone, un groupe alcoxyalkyle contenant 1 à 4 atomes de carbone, un groupe dioxyméthylène, un atome de fluor, un atome de chlore, un atome de brome, un groupe trifluorométhyle, un groupe trifluorométhoxy, un groupe difluorométhoxy, un groupe nitro, un groupe cyano, un groupe azido, un groupe thioalkyle, un groupe sulfonylalkyle contenant chaque fois 1 à 4 atomes de carbone dans le groupe alkyle, un groupe thiobenzyle et un groupe sulfonylbenzyle,

$R^2$ représente un groupe alkyle contenant jusqu'à 8 atomes de carbone, un groupe phényle ou un groupe benzyle,

ou

le radical $OR^8$, $R^8$ représentant un radical d'hydrocarbure saturé ou insaturé à chaîne droite, à chaîne ramifiée ou cyclique contenant jusqu'à 12 atomes de carbone, qui est éventuellement interrompu par un atome d'oxygène ou un atome de soufre dans sa chaîne et/ou qui est éventuellement substitué par un atome d'halogène, un groupe cyano, un groupe hydroxyle, un groupe acétoxy, un groupe phényle, un groupe phénoxy, un groupe phénylthio, un groupe $\alpha$-, $\beta$- ou $\gamma$-pyridyle ou par un groupe amino, ce groupe amino comportant éventuellement un ou deux substituants identiques ou différents choisis parmi le groupe comprenant un groupe alkyle contenant 1 à 4 atomes de carbone, un groupe phényle ou un groupe benzyle,

$R^3$ représente un radical d'hydrocarbure à chaîne droite ou ramifiée contenant jusqu'à 6 atomes de carbone et qui est éventuellement substitué par un ou deux groupes alcoxy contenant 1 à 4 atomes de carbone, ou par un groupe acétoxy,

ou

un groupe phényle ou un groupe benzyle, et

$R^4$, $R^5$, $R^6$ et $R^7$ sont chacun identiques ou différents et représentent un atome d'hydrogène ou un groupe alkyle à chaîne droite ou ramifiée contenant jusqu'à 8 atomes de carbone et qui est éventuellement substitué par un groupe hydroxyle, un groupe methoxy ou un groupe acétoxy, les radicaux $R^4$ et $R^5$, ainsi que $R^6$ et $R^7$ pouvant former, avec l'atome azote correspondant, un noyau pentagonal à heptagonal contenant éventuellement, comme autre hétéro-atome, un atome d'oxygène, un atome de soufre ou un groupe N-alkyle contenant jusqu'à 4 atomes de carbone, ou un groupe N-phényle.

3. Procédé de préparation de composés de formule générale (I) :

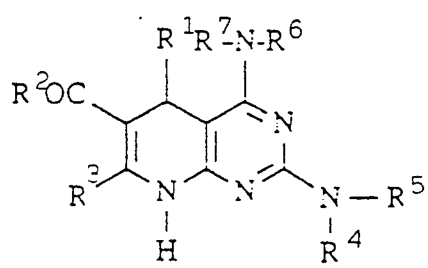

dans laquelle

$R^1$ représente un groupe phényle ou un groupe naphtyle, ou encore un groupe thiényle, un groupe furyle, un groupe pyrryle, un groupe pyrazolyle, un groupe imidazolyle, un groupe oxazolyle, un groupe isoxazolyle, un groupe thiazolyle, un groupe pyridyle, un groupe pyridazinyle, un groupe pyrimidyle, un groupe pyrazinyle, un groupe indolyle, un groupe benzimidazolyle, un groupe benzoxazolyle, un groupe benzisoxazolyle, un groupe benzoxadiazolyle, un groupe benzthiadiazolyle, un groupe quinoléyle, un groupe isoquinoléyle, un groupe quinazolyle ou un groupe quinoxalyle, les hétérocycles mentionnés, de même que le groupe phényle et le groupe naphtyle pouvant comporter chacun 1 à 5 substituants identiques ou différents avec comme substituants le groupe phényle, un groupe alkyle à chaîne droite ou ramifiée contenant jusqu'à 8 atomes de carbone, un groupe cycloalkyle contenant 3 à 7 atomes de carbone, un groupe alcényle ou un groupe alcynyle contenant 2 à 6 atomes de carbone un groupe alcénoxy et un groupe alcynoxy contenant 2 à 6 atomes de carbone, un groupe tri-, tétra- et

pentaméthylène, un groupe dioxyméthylène, un groupe dioxyéthylidène, un atome d'halogène tel qu'un atome de fluor, un atome de chlore, un atome de brome ou un atome d'iode, un groupe trifluorométhyle, un groupe trifluoréthyle, un groupe trifluorométhoxy, un groupe difluorométhoxy, un groupe tétrafluoréthoxy, un groupe dialkylamino contenant 1 à 4 atomes de carbone, un groupe nitro, un groupe cyano, un groupe azido, un groupe alcoxycarbonyle contenant 1 à 4 atomes de carbone dans le groupe alcoxy, un groupe carbamoyle, un groupe N,N-diméthylcarbamoyle, un groupe sulfamoyle, un groupe $SO_m$-alkyle où m représente 0 à 2, et le groupe alkyle contient 1 à 4 atomes de carbone, ou un groupe $SO_m$-benzyle où m représente 0 à 2,

$R^2$ représente un groupe alkyle à chaîne droite, à chaîne ramifiée ou cyclique contenant jusqu'à 8 atomes de carbone ou un groupe phényle ou benzyle,

ou

un groupe amino, un groupe mono- ou dialkylamino contenant jusqu'à 4 atomes de carbone par groupe alkyle, un ou les deux groupes alkyle pouvant être substitués par un groupe phényle,

ou

un groupe anilino qui est éventuellement substitué par un atome de fluor ou un atome de chlore, ou par un groupe alkyle ou un groupe alcoxy contenant 1 à 4 atomes de carbone,

ou

le radical $OR^8$, $R^8$ représentant un radical d'hydrocarbure saturé ou insaturé à chaîne droite, à chaîne ramifiée ou cyclique contenant jusqu'à 12 atomes de carbone et éventuellement interrompu dans sa chaîne par un atome d'oxygène ou un atome de soufre et/ou éventuellement substitué par un atome d'halogène, un groupe cyano, un groupe hydroxyle, un groupe acétoxy, un groupe benzoyloxy, un groupe nitro, un groupe nitrooxy, un groupe alcoxycarbonyle contenant jusqu'à 4 atomes de carbone dans le groupe alcoxy, un groupe phényle, un groupe phénoxy, un groupe phenylthio, un groupe phénylsulfonyle, un groupe α-, β- ou γ-pyridyle ou encore un groupe amino, ce groupe amino comportant éventuellement un ou deux substituants identiques ou différents choisis parmi le groupe comprenant un groupe alkyle contenant 1 à 4 atomes de carbone, un groupe alcoxyalkyle contenant jusqu'à 6 atomes de carbone, un groupe phényle, un groupe benzyle ou un groupe phénéthyle, l'atome d'azote de ce groupe amino formant éventuellement, avec les substituants, un noyau pentagonal à heptagonal qui, comme autre hétéro-atome, peut contenir un atome d'oxygène ou un atome de soufre, ou encore un groupe N-phényle ou un groupe N-alkyle ayant 1 à 4 atomes de carbone dans le radical alkyle,

$R^3$ représente un atome d'hydrogène,

ou

un radical d'hydrocarbure à chaîne droite, à chaîne ramifiée ou cyclique contenant jusqu'à 6 atomes de carbone et éventuellement substitué par un ou deux groupes alcoxy contenant 1 à 4 atomes de carbone, ou par un groupe acétoxy ou benzoyloxy,

ou

un groupe phényle où un groupe benzyle,

et

$R^4$, $R^5$, $R^6$ et $R^7$ sont identiques ou différents et représentent chacun un atome d'hydrogène

ou

un groupe alkyle à chaîne droite ou ramifiée éventuellement substitué par un groupe hydroxyle, un groupe méthoxy, un groupe acétoxy ou un groupe benzoyloxy et contenant jusqu'à 8 atomes de carbone, les radicaux $R^4$, $R^5$, ainsi que $R^6$ et $R^7$ pouvant former, avec l'atome azote correspondant, un noyau pentagonal à heptagonal contenant éventuellement, comme autre hétéro-atome, un atome d'oxygène ou un atome de soufre, ou encore un groupe N-alkyle ayant jusqu'à 4 atomes de carbone, ou un groupe N-phényle,

ou

un groupe phényle ou un groupe benzyle,

caractérisé en ce que :

A) on fait réagir des composés d'ylidène-β-dicarbonyle de formule générale (II) :

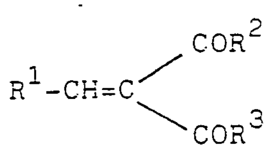

dans laquelle

$R^1$, $R^2$ et $R^3$ ont les significations indiquées dessus, avec des 6-aminopyrimidines de formule générale (III) :

(Voir Formule page 42)

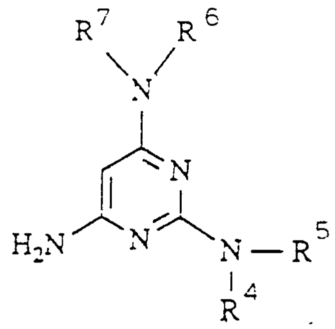

dans laquelle

$R^4$, $R^5$, $R^6$ et $R^7$ ont les significations indiquées ci-dessus, éventuellement en présence de solvants organiques,

ou

B) on fait réagir des aldéhydes de formule générale (IV) :

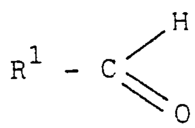

dans laquelle

$R^1$ a la signification indiquée ci-dessus, avec des composés β-dicarbonyle de formule générale (V) :

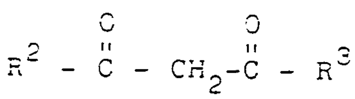

dans laquelle

$R^2$ et $R^3$ ont les significations indiquées ci-dessus, et des 6-aminopyrimidines de formule générale (III) :

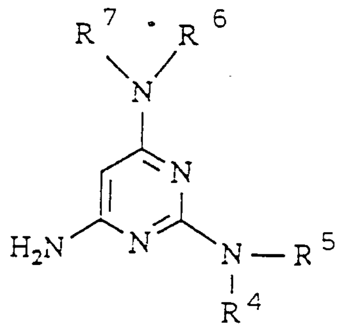

dans laquelle

$R^4$, $R^5$, $R^6$ et $R^7$ ont les significations indiquées ci-dessus, éventuellement en présence de solvants organiques inertes, à des températures comprises entre 20 et 150 °C.

4. Composés de formule générale (I) selon la revendication 1, en vue de les utiliser pour combattre les maladies de la circulation.

5. Médicament contenant au moins un composé répondant à la formule générale (I) selon la revendication 1.

6. Procédé de préparation de médicaments, caractérisé en ce qu'on transforme, en une forme d'application appropriée, au moins un composé de formule générale (I) selon la revendication 1 en utilisant éventuellement des substances auxiliaires et des substances supports.

7. Utilisation de composés de formule générale (I) selon la revendication 1 lors de la préparation d'agents destinés à la circulation.